(19) Europäisches Patentamt
European Patent Office
Office européen des brevets

(11) Veröffentlichungsnummer: **0 475 443 B1**

(12) # EUROPÄISCHE PATENTSCHRIFT

(45) Veröffentlichungstag der Patentschrift: **19.10.94**

(51) Int. Cl.[5]: **C07C 317/36**, C07C 315/04, C07H 21/00

(21) Anmeldenummer: **91115583.6**

(22) Anmeldetag: **13.09.91**

(54) **Verfahren zur chemischen Synthese von Oligonukleotiden.**

(30) Priorität: **14.09.90 DE 4029244**
**09.04.91 DE 4111363**

(43) Veröffentlichungstag der Anmeldung:
**18.03.92 Patentblatt 92/12**

(45) Bekanntmachung des Hinweises auf die Patenterteilung:
**19.10.94 Patentblatt 94/42**

(84) Benannte Vertragsstaaten:
**AT BE CH DE DK ES FR GB GR IT LI LU NL SE**

(56) Entgegenhaltungen:
**EP-A- 0 267 996**
**EP-A- 0 272 928**
**DE-A- 1 953 600**

**NUCLEIC ACIDS RESEARCH. Bd. 17, Nr. 7, 11. April 1989, ARLINGTON, VIRGINIA US Seiten 2379 - 2390; C.LEHMANN ET AL.: 'Solid-phase Synthesis of Oligoribonucleotides Using 9-Fluorenylmethoxycarbonyl (Fmoc) for 5'-Hydroxyl Protection'**

**BIOORGANIC CHEMISTRY Bd. 14, 1986, Seiten 274 - 325; E.SONVEAUX: 'The Organic Chemistry Underlying DNA Synthesis'**

(73) Patentinhaber: **HOECHST AKTIENGESELLSCHAFT**

**D-65926 Frankfurt (DE)**

(72) Erfinder: **Pfleiderer, Wolfgang, Prof.Dr.**
**Lindauer Strasse 47**
**W-7750 Konstanz (DE)**
Erfinder: **Bergmann, Frank**
**Im Baumgarten 2**
**W-8995 Weissenberg (DE)**

**EP 0 475 443 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die chemische Polykondensation von Mononukleotiden ist eine wichtige Methode zur Herstellung von Desoxyribonukleinsäure (DNA) oder Ribonukleinsäure (RNA).

Ein grundlegendes Problem bei der chemischen Synthese von DNS oder RNS ist das Auffinden geeigneter Schutzgruppen der Amino- und Hydroxy-Gruppen der Nukleosid-Basen und der Zuckerreste. Diese Schutzgruppen müssen einerseits unter den Bedingungen der Polykondensationreaktion, d.h. während der Bildung der Phosphodiester-Bindung, stabil sein und andererseits müssen sie genügend labil sein, um am Ende der Reaktion, ohne die Phosphodiester-Bindung zu spalten, wieder entfernt werden zu können (H.G. Khorana; Pure Appl. Chem. 17 (1968) 349).

Besonders problematisch ist die chemische Synthese von RNA, da der Ribose-Zuckerrest zwei Hydroxy-Gruppen trägt, die beide geschützt werden müssen. Die Schutzgruppe der 5'-Hydroxy-Gruppe muß dabei vor jedem Polykondensationsschritt selektiv, d.h. ohne Abspaltung der 2'-Hydroxy-Schutzgruppe wieder abgespalten werden. Die Schutzgruppe der 2'-Hydroxy-Gruppe hingegen darf erst am Ende der RNA-Synthese abgespalten werden, und zwar unter Bedingungen, die zu keiner Spaltung oder Isomerierung der Phosphodiesterbindungen führen (C.B. Reese, Nucleic Acids and Molecular Biology, Vol 3 (F. Eckstein & D.M.J. Lilley eds.) Springer-Verlag, Weinheim).

Eine Möglichkeit der selektiven Abspaltung der 5'-Hydroxy-Schutzgruppe ohne Abspaltung der 2'-Hydroxy-Schutzgruppe ist durch die Kombination einer basenlabilen 5'-Hydroxy-Schutzgruppe mit einer säurelabilen 2'-Hydroxy-Schutzgruppe zu erreichen (Chr. Lehmann et al. (1989) Nucleic Acids Res. 17, 2379-2390, No. 7). Die Verwendung einer basenlabilen 5'-Hydroxy-Schutzgruppe ist auch bei der Synthese von DNA von Vorteil, da unter den milden nicht sauren Hydrolysebedingungen die in der Synthese bereits gebildeten Phosphodi- bzw. triesterbindungen im allgemeinen nicht gespalten werden. Zudem wird unter den milden nicht-sauren Hydrolysebedingungen eine Depurinierung der Nukleotide, wie sie bei E. Sonveaux (E. Sonveaux (1986), Bioorganic Chemistry 14, 286) beschrieben ist, umgangen. Eine weitere Anforderung an die 5'-Hydroxy-Schutzgruppe bei der DNA-, wie auch bei der RNA-Synthese, ist eine leichte und hochempfindliche Nachweisbarkeit der Schutzgruppe. Auf diese Weise kann der Umsetzungsgrad der einzelnen Reaktionsschritte besonders gut verfolgt und eine möglichst vollständige Umsetzung erreicht werden. Dadurch ist es möglich, besonders lange Oligonukleotide mit hoher Ausbeute herzustellen. Dies erlaubt auch die Ausführung kleiner Synthese-Ansätze im nanomolaren bis picomolaren Bereich.

Es wurde nun überraschenderweise gefunden, daß die Dansylethoxycarbonylgruppe (Dans-EOC) als basenlabile 5'-Hydroxy-Schutzgruppe in der chemischen Oligonukleotidsynthese eingesetzt werden kann.

Gegenstand der Erfindung ist somit

1. ein Verfahren zur Herstellung der Verbindung der Formel (I),

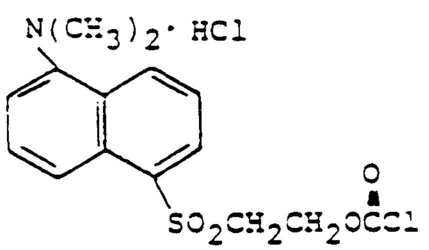

(I),

dadurch gekennzeichnet, daß die Verbindung der Formel (II),

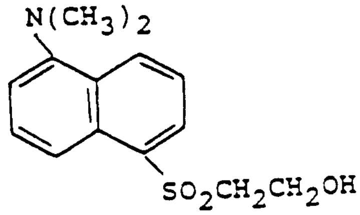

(II),

mit einem Chlorcarbonyl-Donor umgesetzt wird.

2. eine Verbindung der Formel (IIIa) oder (IIIb)

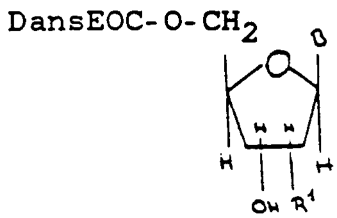

(IIIa)

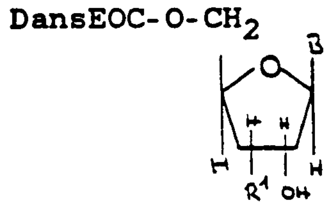

(IIIb)

worin bedeuten:

R¹ Wasserstoff oder unabhängig voneinander

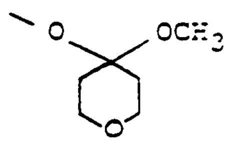

eine Gruppe der Formel

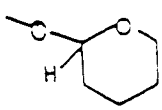

,

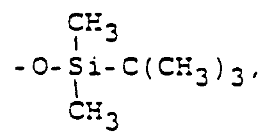

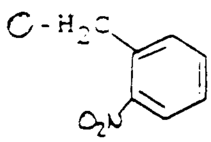

,

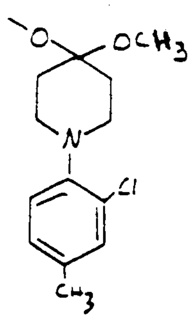

oder

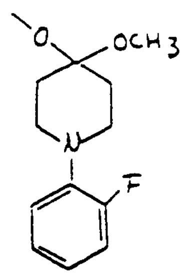

und

B bedeutet

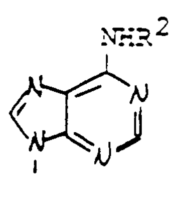

,

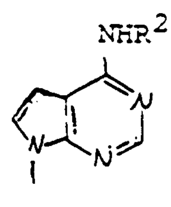

,

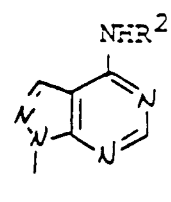

,

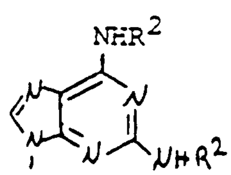

oder

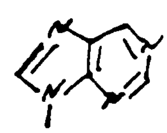

mit $R^2$ jeweils unabhängig voneinander eine Gruppe der Formel

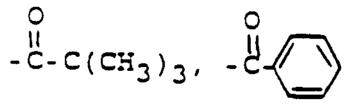

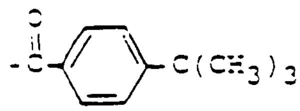 oder 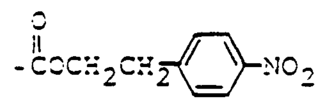

oder

B bedeutet

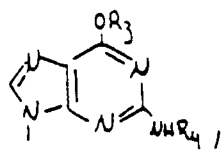 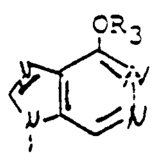 oder 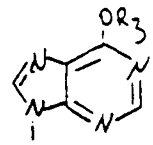

mit $R^3$ Wasserstoff oder

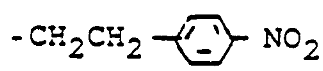

und

$R^4$

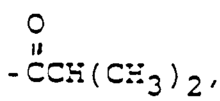 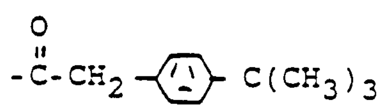 oder

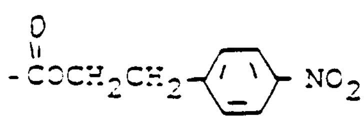

oder

B bedeutet

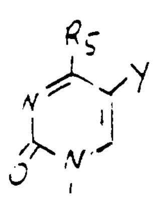 oder 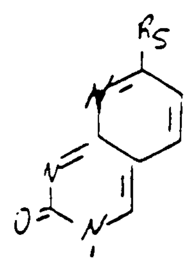

mit $R_5$ -OH,

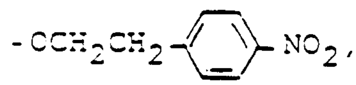

Y = H, Alkyl ($C_1$-$C_4$) insbesondere $CH_3$

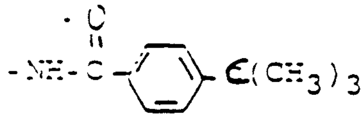 oder 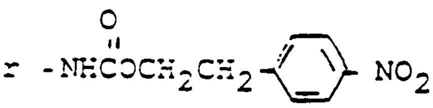

3. ein Verfahren zur Herstellung einer Verbindung der Formel (IIIa) oder (IIIb) durch Umsetzung der Verbindung der Formel (I) mit einer entsprechenden Verbindung der Formel (IVa) oder (IVb)

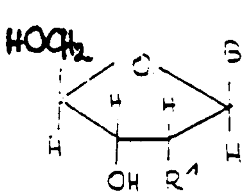 (IVa) 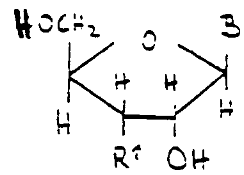 (IVb)

worin $R^1$ und B die obige Bedeutung haben, in Gegenwart einer Base, vorzugsweise Pyridin oder einer Mischung bestehend aus Tetrahydrofuran, Dioxan, Methylenchlorid, Chloroform und/oder Acetonitril und einer Verbindung der Formel

$NR^{20}R^{21}R^{22}$

worin $R^{20}$, $R^{21}$ und $R^{22}$ gleich oder unabhängig verschieden Wasserstoff oder die $C_1$-$C_4$-Alkyl-, vorzugsweise eine Trimethyl-, Triethyl- oder Diisopropyl-Gruppe bedeuten.

4. eine Verbindung der Formel (Va) oder (Vb)

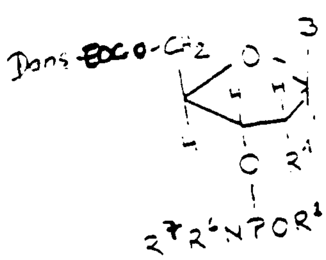 (Va) 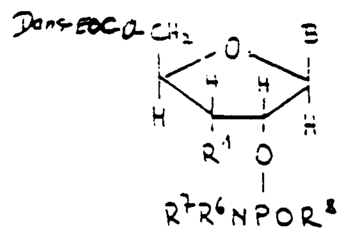 (Vb)

worin

DansEOC, $R^1$ und B die obengenannte Bedeutung haben und $R^6$, $R^7$ gleich oder unabhängig verschieden eine $C_1$-$C_8$-Alkyl-, vorzugsweise eine Isopropyl- oder $C_5$-$C_{12}$-Cycloalkyl-Gruppe, vorzugsweise bis $C_8$, Benzyl oder Phenyl oder zusammen mit dem Stickstoffatom, an das sie gebunden sind, einen gesättigten oder ungesättigten heterocyclischen Ring, der gegebenenfalls weitere Heteroatome und Substituenten enthalten kann und $R^8$ eine Gruppe der Formel

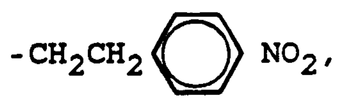 , 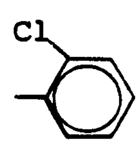

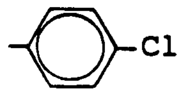

oder $CH_3$,
oder eine Benzylgruppe, welche nicht substituiert oder einfach oder mehrfach ringsubstituiert, vorzugsweise nicht substituiert ist, wobei der oder die Substituenten unabhängig voneinander ein Halogen, eine $C_1$-$C_4$-Alkyl-,Nitro-, Methoxy- oder Carboxylgruppe ist.
5. ein Verfahren zur Herstellung einer Verbindung der Formel (Va) oder (Vb) durch Umsetzung der Verbindung der Formel (IIIa) oder (IIIb) mit einer Verbindung der Formel (VI)

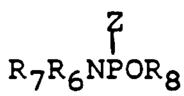 (VI),

worin $R_6$, $R_7$ und $R_8$ die obige Bedeutung haben und Z Chlor oder Brom oder ein Rest der Formel $-NR_9R_{10}$, wobei für $R_9$ und $R_{10}$ unabhängig voneinander dieselben Reste infrage kommen wie für $R_6$, wenn Z gleich Chlor ist, in Gegenwart einer Base, vorzugsweise Pyridin oder einer Mischung von Tetrahydrofuran, Dioxan, Methylenchlorid, Chloroform und/oder Acetonitril mit einem $C_1$-$C_4$-Trialkylamin, vorzugsweise einem Trimethyl-, Triethyl- oder Diisopropylethyl-Amin oder wenn Z ein Rest der Formel $-NR_9R_{10}$ ist, dann in Gegenwart einer Verbindung der Formel $[HNR_{11}R_{12}R_{13}]^{(+)}X^{(-)}$, wobei $R_{11}$, $R_{12}$, $R_{13}$ gleich oder unabhängig verschieden eine $C_1$-$C_4$-Alkyl-Gruppe und X = Halogen, insbesondere Chlor bedeuten, oder Tetrazol, vorzugsweise in Gegenwart von Tetrazol.
6. ein Verfahren zur Herstellung von Oligonukleotiden aus Verbindungen der Formel (Va) und/oder (Vb), dadurch gekennzeichnet, daß man eine Verbindung der Formel (Va) oder (Vb)
1. mit einer Verbindung der Formel (VIIa) oder (VIIb)

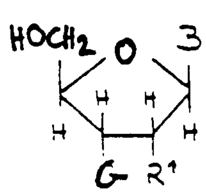 (VIIa) oder 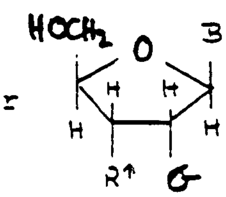 (VIIb)

worin B und $R^1$ die obengenannte Bedeutung haben und G gleichbedeutend mit $R^1$ oder ein polymerer Träger ist, der über die 2'-Hydroxy- oder 3'-Hydroxy-Gruppe der Verbindung der Formel (VIIa) oder (VIIb) gebunden ist, umsetzt,
2. die erhaltenen Verbindungen oxidiert
3. die Dansylethoxycarbonyl-Gruppe abspaltet,
4. die erhaltene Verbindung mit einer Verbindung der Formel (Va) oder (Vb) umsetzt und
5. die Reaktionsschritte 2-4 bis zur gewünschten Kettenlänge wiederholt.

7. eine Verbindung der Formel (VIIIa) oder (VIIIb)

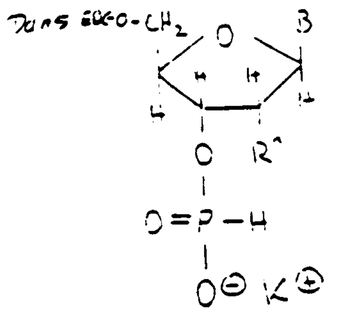

(VIIIa)

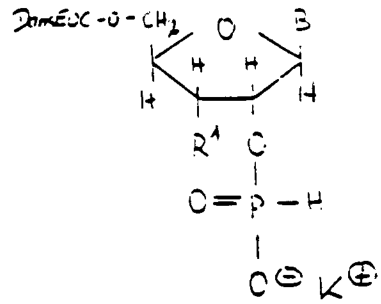

(VIIIb)

worin DansEOC, $R^1$ und B die obengenannte Bedeutung haben und $K^{(+)}$ ein Kation, insbesondere $[HN-(C_2H_5)_3]^{(+)}$ ist.

8. ein Verfahren zur Herstellung einer Verbindung der Formel (VIIIa) oder (VIIIb) durch Umsetzung einer Verbindung der Formel (IIIa) oder (IIIb) mit einer Verbindung der Formel (IX)

$PR_{14}R_{15}R_{16}$ (IX)

worin $R_{14}$, $R_{15}$, $R_{16}$ gleich oder unabhängig verschieden Wasserstoff oder eine $C_1$-$C_8$-Alkyl-, $C_1$-$C_8$-Fluoralkyl-, oder Aryl-Gruppe vorzugsweise eine 2,2,2-Trifluoroethyl-, 1,1,1,3,3,3,-Hexafluoro-2-propyl-, Ethyl- oder Phenyl-Gruppe in Gegenwart einer Base.

9. ein Verfahren zur Herstellung einer Verbindung der Formel (VIIIa) oder (VIIIb) durch Umsetzung einer Verbindung der Formel (IIIa) oder (IIIb) mit einer Verbindung der Formel (X)

$PR_{17}R_{18}R_{19}$ (X)

worin $R_{17}$, $R_{18}$, $R_{19}$ gleich oder unabhängig verschieden Chlor, Brom oder eine $C_1$-$C_8$-Alkylamino- oder 1,2,4-Triazolylgruppe, vorzugsweise eine 1,2,4-Triazolylgruppe, in Gegenwart einer Base mit anschließender Hydrolyse.

10. ein Verfahren zur Herstellung von Oligonukleotiden aus Verbindungen der Formel (VIIIa) und/oder (VIIIb), dadurch gekennzeichnet, daß man eine Verbindung der Formel (VIIIa) oder (VIIIb)

1. mit einer Verbindung der Formel (VIIa) oder (VIIb) umsetzt,
2. die Dansylethoxycarbonyl-Gruppe abspaltet,
3. die erhaltene Verbindung mit einer Verbindung der Formel (VIIIa) oder (VIIIb) umsetzt,
4. die Reaktionsschritte 2 und 3 bis zur gewünschten Kettenlänge wiederholt und
5. das erhaltene Oligonukleotid oxidiert.

Zur Einführung der Dansylethoxycarbonylgruppe in das Nukleosid wurde 2-Dansylethylchloroformiat-Hydrochlorid mit einem Nuleosid umgesetzt, bei dem je nach Nukleosid die Amino- bzw. Hydroxy-Funktionen der Nukleosidbase durch geeignete Gruppen geschützt sind. Als Schutzgruppen eignen sich beispielsweise für die 6-Aminogruppe von Adenin die t-Butylcarbonyl, Benzoyl-, 4-(t-Butyl)-benzoyl- oder para-Nitrophenylethyloxycarbonyl-Gruppe, insbesondere die Benzoyl- oder die para-Nitrophenylethyloxycarbonyl-Gruppe.

Für die 2-Aminogruppe von Guanin eignen sich beispielsweise die Isobutyryl-, 4-(t-Butyl)-phenylacetyl- oder para-Nitrophenylethyloxycarbonyl-Gruppe, insbesondere die Isobutyryl- oder die para-Nitrophenylethyloxycarbonyl-Gruppe. Die 6-Hydroxy-Gruppe von Guanin bzw. die 4-Hydroxy-Gruppe von Uracil bleiben im allgemeinen entweder ungeschützt oder sie werden durch eine para-Nitrophenylethyl-Gruppe geschützt. Bei Cytosin wird die 4-Amino-Gruppe beispielsweise durch eine Benzoyl-, 4-(t-Butyl)-benzoyl- oder para-Nitrophenylethyloxycarbonyl-Gruppe, insbesondere die Benzoyl- oder die para-Nitrophenylethyloxycarbonyl-Gruppe geschützt. Thymidin bleibt im allgemeinen ungeschützt. Bei Uridin wird die 3-N-Gruppe beispielsweise durch eine Boc- oder Anisoyl-Gruppe geschützt.

Anstelle der natürlichen Nukleosid-Basen können auch modifizierte Nukleosid-Basen verwendet werden, deren Amino- bzw. Hydroxy-Gruppen in analoger Weise durch die oben erwähnten Schutzgruppen geschützt werden können. Beispiele für Nucleoside mit modifizierten Basen sind Inosin-, 8-Aza-7-deazaadenosin-, Tubercidin-, Nebularin-, Xanthosin-, 2-Aminoadenosin- oder Pyridopyrimidin-Nucleoside. Die Nukleoside sind käuflich und die Einführung der einzelnen Schutzgruppen kann beispielsweise nach C. Lehmann et al. (1989), C.B. Reese (1989) ["The Chemical Synthesis of Oligo and Polyribonucleotides" in Nucleic Acids and Molecular Biology 3, F. Eckstein & D.M.J. Lilley (eds.), Springer Verlag Berlin, Heidelberg], E. Sonveaux (1986), Bioorganic Chemistry 14, 274-325 oder E. Uhlmann & A. Peyman (1990), Chemical Reviews 90, 543-584, No. 4 erfolgen.

Bei der Verwendung von Ribonukleotiden muß zusätzlich zu der Hydroxy- und Aminogruppe der Nukleotidbasen auch die 2'-Hydroxygruppe des Riboserestes geschützt werden. Wie bereits erwähnt, ist es für die RNS-Synthese wichtig, durch die Wahl einer geeigneten Kombination von 5'-Hydroxy- und 2'-Hydroxyschutzgruppe die 5'-Hydroxyschutzgruppe selektiv, d.h. ohne Abspaltung der 2'-Hydroxyschutzgruppe, entfernen zu können.

In Anwesenheit von säurelabilen 2'-Hydroxyschutzgruppen, kann nun die Dansylethoxycarbonylgruppe als 5'-Hydroxyschutzgruppe selektiv unter nicht-sauren Bedingungen abgespalten werden. Als säurelabile 2'-Hydroxyschutzgruppen können beispielsweise die 4-Methoxytetrahydropyran-4-yl-, Tetrahydropyranyl-, t-Butyl-dimethylsilyl-, 2-Nitrobenzyl-, 1-(2-Chloro-4-methylphenyl)-4-methoxypiperidin-4-yl-, oder die 1-(2-Fluorophenyl)-4-methoxypiperidin-4-yl-Gruppe verwendet werden. Die Abspaltung der Dansylethoxycarbonylgruppe wird vorzugsweise in einem aprotischen polaren Lösemittel, insbesondere Acetonitril oder Pyridin mit Hilfe von 1 bis 3, vorzugsweise 1,5 bis 2,5 Moläquivalente DBU (= 1,5-Diazabicyclo-[5.4.0]-undec-5-en) durchgeführt. Alternativ können andere Basen wie TMG (= $N^1N^1N^2N^2$-Tetramethylguanidin) oder $C_1$-$C_4$-Trialkylamine, wie beispielsweise Triethylamin zur Abspaltung eingesetzt werden.

2-Dansylethylchloroformiat-Hydrochlorid als Ausgangsverbindung der 5'-Hydroxy-Schutzgruppe des Ribose- bzw. Desoxyriboserestes wurde durch Umsetzung von 2-Dansylethanol mit einem Chlorcarbonyl-Donor, wie beispielsweise Trichlormethylchloroformiat (Diphosgen) und/oder Phosgen, vorzugsweise Trichlormethylchloroformiat in Gegenwart eines polaren, aprotischen Lösemittels hergestellt. In einer bevorzugten Ausführungsform wurde die Reaktion in Gegenwart eines einzigen polaren, aprotischen Lösemittels, insbesondere in Gegenwart von Acetonitril, durchgeführt. Das Molverhältnis von 2-Dansylethanol zu dem ChlorcarbonylDonor war 0,5-1 zu 1-2, vorzugsweise 1 zu 1-2, insbesondere 1 zu 1,5-2. Die Reaktionstemperatur lag in einem Bereich von -20 °C bis zum Siedepunkt der Reaktionsmischung, vorzugsweise von -5 °C bis +20 °C, insbesondere von 0 °C bis 5 °C.

Nach dem erfindungsgemäßen Verfahren fällt das 2-Dansylethylchloroformiat-Hydrochlorid als ein reines und durch die Elementaranalyse in seiner Zusammensetzung bestätigtes Produkt an. Dies ist deshalb so überraschend, weil A. Takadate et al. (A. Takadate et al. (1983) Yakugaku Zasshi 103, 982-966) durch Umsetzung von 2-Dansylethanol mit Trichlormethylchloroformiat ein Produkt erhielten, dessen Schmelzpunkt um ca. 20 °C niedriger ist als das in dem genannten Verfahren synthetisierte Produkt. 2-Dansylethanol kann beispielsweise nach S. Goya et al. (S. Goya et al (1981) Yakugaku Zasshi 101, 1164) hergestellt werden.

Die Umsetzung von 2-Dansylethylchloroformiat-Hydrochlorid mit dem geschützten Nukleosid kann beispielsweise analog der Umsetzung mit 9-Fluorenylmethoxycarbonylchlorid nach C. Lehmann et al. in Gegenwart einer Base durchgeführt werden. Als Base eignen sich organische Basen, insbesondere Pyridin oder einer Mischung bestehend aus Tetrahydrofuran, Dioxan, Methylenchlorid, Chloroform und/oder Acetonitril und einer Verbindung der Formel

$NR^{20}R^{21}R^{22}$

worin $R^{20}$, $R^{21}$ und $R^{22}$ gleich oder unabhängig verschieden Wasserstoff oder eine $C_1$-$C_4$-Alkyl-, vorzugsweise eine Trimethyl-, Triethyl- oder Diisopropyl-Gruppe bedeuten. Wird dabei als Substrat ein 2'-geschütztes Ribonukleosid verwendet, so entsteht ein Produktgemisch bestehend aus dem Dansylethyloxycarbonyl-Ribonukleosid und dem Bis-Dansylethyloxycarbonyl-Ribonukleosid als Nebenprodukt. Dieses Produktgemisch kann direkt in der nachfolgenden Phosphorylierungsreaktion eingesetzt werden. Vorzugsweise kann das Gemisch auch beispielsweise mittels Flash-Chromatographie gereinigt werden. Das abgetrennte Bis-Dansylethyloxycarbonyl-Ribonukleosid kann dann anschließend beispielsweise mit DBU in das Dansyl-freie Ribonukleosid gespalten werden, das wiederum als Ausgangsverbindung für die Dansylierungsreaktion verwendet werden kann.

Zum Aufbau 2',5'-verknüpfter Oligoribonucleotide, welche zum Beispiel als Tri- oder Tetraadenylat die Proteinbiosynthese inhibieren (Kerr, I.M. & Brown, R.E. (1978) Proc. Natl. Acad. Sci. USA. 75, 256-260), kann man ein entsprechend der obigen Beschreibung geschütztes Nukleosid mit einer freien 2'-Hydroxygruppe in analoger Weise mit 2-Dansylethylchloroformiat-Hydrochlorid umsetzen.

Das dansylierte Nukleosid mit der noch freien 2'- oder 3'-Hydroxygruppe am Zuckerrest wird im allgemeinen phosphityliert. Als Phosphitylierungsreagenz kann beispielsweise eine Verbindung der Formel

(VI)

$$R_7R_6N\overset{Z}{P}OR_8 \qquad (VI),$$

verwendet werden, worin bedeuten:

$R_6$, $R_7$ gleich oder unabhängig verschieden $C_1$-$C_8$-Alkyl-, vorzugsweise eine Isopropyl- oder $C_5$-$C_{12}$-Cycloalkyl-Gruppe, vorzugsweise bis $C_8$, eine Benzyl oder eine Phenyl-Gruppe oder zusammen mit dem Stickstoffatom, an das sie gebunden sind, einen gesättigten oder ungesättigten heterocyclischen Ring, der gegebenenfalls weitere Heteroatome und Substituenten enthalten kann

$R_8$ eine Gruppe der Formel

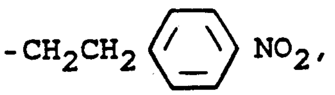, 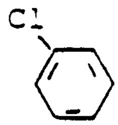, 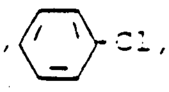,

oder $-CH_3$

oder eine Benzylgruppe, welche nicht substituiert oder einfach oder mehrfach ringsubstituiert, vorzugsweise nicht substituiert ist, wobei der oder die Substituenten unabhängig voneinander beispielsweise ein Halogen, eine $C_1$-$C_4$-Alkyl-, Nitro-, Methoxy- oder Carboxylgruppe ist,

Z Chlor, Brom oder ein Rest der Formel $-NR_9R_{10}$, wobei $R_9$, $R_{10}$ gleich oder unabhängig verschieden eine $C_1$-$C_8$-Alkyl-, vorzugsweise eine Isopropyl- oder $C_5$-$C_{12}$-Cycloalkyl-Gruppe, vorzugsweise bis $C_8$, eine Benzyl- oder eine Phenyl-Gruppe bedeuten.

Vorzugsweise wurde als Phosphitylierungsreagenz eine Verbindung der Formel (VII) mit Z = Chlor, $R_6$ und $R_7$ je ein Isopropylrest und $R_8$ gleich eine Gruppe der Formel

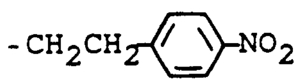

verwendet.

Die Reaktion wurde im allgemeinen in einem organischen Lösemittel wie Tetrahydrofuran oder Methylenchlorid, vorzugsweise Methylenchlorid, in Anwesenheit von 1 bis 8, vorzugsweise 1 bis 6, insbesondere 1 bis 4 Moläquivalente einer organischen Base wie Pyridin oder eine Mischung von Tetrahydrofuran (THF), Dioxan, Methylenchlorid, Chloroform und/oder Acetonitril und einem $C_1$-$C_4$-Trialkylamin, vorzugsweise einem Trimethyl-, Triethyl- oder Diisopropylethyl-Amin, insbesondere Diisopropylethyl-Amin durchgeführt. Ist Z ein Rest der Formel $-NR_9R_{10}$, so wurde die Reaktion vorzugsweise in Anwesenheit einer Verbindung der Formel $[HNR_{11}R_{12}R_{13}]^{(+)}X^{(-)}$, wobei $R_{11}$, $R_{12}$, $R_{13}$ gleich oder unabhängig verschieden eine $C_1$-$C_4$-Alkyl-Gruppe und $X^{(-)}$ = Halogenid, insbesondere ein Chlorid, oder ein Tetrazolid bedeuten, oder Tetrazol, vorzugweise in Gegenwart von Tetrazol, durchgeführt. Das Molverhältnis von dansyliertem Nukleosid zu Phosphitylierungsreagenz betrug 1 zu 1 - 4 vorzugsweise 1 zu 2 - 4, insbesondere 1 zu 2,5 - 3,5.

Die so gewonnenen Verbindungen der Formel (Va) oder (Vb) können anschließend für die Oligonukleotidsynthese eingesetzt werden. Hierbei sind die Zuckerreste der Nukleotide für die DNA-Synthese Desoxyribose, für die RNA-Synthese Ribose, aber es können auch Gemische aus Desoxyribose und Ribose für die Synthese eines Oligonukleotids bestehend aus regelmäßig oder unregelmäßig angeordneten Desoxyribose- und Ribosezuckerresten sein. Ferner kann das Oligonukleotid regelmäßig oder unregelmäßig aus Mononukleotiden der Formel (Va) und (Vb) aufgebaut sein. Die Oligo- bzw. Polynukleotidsynthese kann auf analoger Weise nach der Phosphoramidit-Methode, wie beispielsweise bei Chr. Lehmann et al. (1989) beschrieben, durchgeführt werden.

Grundsätzlich gibt es zwei Möglichkeiten, Oligonukleotide zu synthetisieren . Zum einen kann die Synthese in Lösung erfolgen, beispielsweise nach der von C.B. Reese beschriebenen Methode (C.B. Reese (1989) "The Chemical Synthesis of Oligo- and Poly-ribonucleotides" in Nucleic Acids and Molecular Biology (F. Eckstein & D.M.J. Lilley, eds.) 3, 164-181).

Zum anderen kann die Oligonukleotid-Synthese an der Festphase, beispielsweise an Nukleosidfunktionalisierten Glass, erfolgen (K.P. Stengele & W. Pfleiderer (1989) Nucleic Acids Res. Symp. Ser. 21, 101, K.P. Stengele & W. Pfleiderer (1990) Tetrahedron Lett. 31, 2549 oder Chr. Lehmann et al. (1989) Nucleic Acids Res. 17, 2379-2390, No. 7). Im allgemeinen ist die Festphasensynthese die bevorzugte Methode.

Hierbei wurde vorzugsweise die folgende Reaktionsfolge gewählt:

1. Umsetzung einer Verbindung der Formel (Va) oder (Vb) mit dem Nukleosid der Formel (VIIa) oder (VIIb)

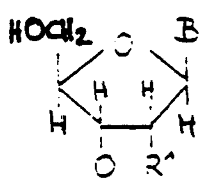

(VIIa) oder

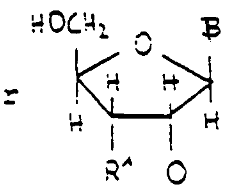

(VIIb)

worin B und $R^1$ die obengenannte Bedeutung haben, und G gleichbedeutend mit $R_1$ oder ein polymerer Träger ist, der über die 2'-Hydroxy- oder 3'-Hydroxy-Gruppe der Verbindung der Formel (VIIa) oder (VIIb) gebunden ist, in Anwesenheit einer schwachen Säure, beispielsweise Tetrazol oder p-Nitrophenyl-tetrazol.

2. Abfangen nicht-umgesetzter Verbindungen der Formel (VIIa) oder (VIIb), beispielsweise mit Acetanhydrid.

3. Oxidation zum Phosphat, Phosphoramidat oder zum Thiophosphat, beispielsweise mit Jod, Schwefel oder Jod/Amin.

4. Abspaltung der Dansylethoxycarbonyl-Gruppe beispielsweise mit DBU in Acetonitril.

5. Umsetzung der erhaltenen Träger-gebundenen Verbindung mit einer Verbindung der Formel (Va) oder (Vb).

6. Wiederholung der Reaktionsschritte 2 bis 6 zur gewünschten Kettenlänge des Oligonukleotids.

Die Verbindungen der Formel (Va) oder (Vb) und (VIIa) oder (VIIb) wurden vorzugsweise bei -20 bis +100 °C, insbesondere bei Raumtemperatur in Anwesenheit von beispielsweise Tetrazol oder para-Nitrophenyl-tetrazol als schwache Säuren umgesetzt. Die Oxidation erfolgte bei einer Temperatur von -80 bis 100 °C, vorzugsweise bei -20 bis +60 °C, in Anwesenheit von Jod, Schwefel oder Jod in Gegenwart eines Amins (A. Jäger et al. Biochemistry 27, 7237 (1988)). Wurde ein Gemisch aus Jod, Wasser und einer organischen Base wie Lutidin oder Pyridin verwendet, so erfolgte die Oxidation vorzugsweise bei Raumtemperatur. Wurde hingegen ein Gemisch aus elementarem Schwefel, Toluol und einer organischen Base verwendet, so erfolgte die Oxidation vorzugsweise bei 60 °C.

Die synthetisierten Oligonukleotide bestanden im allgemeinen aus 2 bis ca. 200, vorzugsweise 2 bis 100, insbesondere 2 bis 20 Mononukleotiden.

Alternativ zur Phosphitylierung kann das dansylierte Nukleosid mit der noch freien 2'- oder 3'-Hydroxygruppe am Zuckerrest auch zum H-Phosphonat der Formel (VIIIa) oder (VIIIb) umgesetzt werden

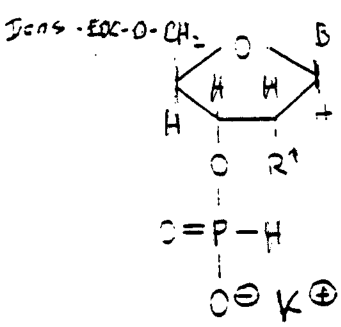

(VIIIa)

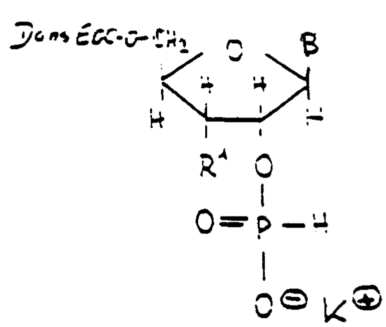

(VIIIb)

worin DansEOC, $R^1$ und B die obengenannte Bedeutung haben und $K^{(+)}$ ein Kation, insbesondere $[NH(C_2H_5)_3]^{(+)}$, ist (R. Strömberg, Chem. Commun. 1987, 1; B.C. Froehler, P.G. Ng, M.D. Matteucci, Nucleic Acids Res. 14 (1986) 5399; M. Tahalu et al., Chem. Lett. 1988, 1675).

Dabei wird im allgemeinen eine Verbindung der Formel (IIIa) oder (IIIb) mit einer Verbindung der Formel (IX) oder (X)

$PR_{14}R_{15}R_{16}$ (IX)

$PR_{17}R_{18}R_{19}$ (X)

worin $R_{14}$, $R_{15}$, $R_{16}$ gleich oder unabhängig verschieden Wasserstoff oder eine $C_1$-$C_8$-Alkyl-, $C_1$-$C_8$-Fluoralkyl- oder Aryl-Gruppe und $R_{17}$, $R_{18}$, $R_{19}$ gleich oder unabhängig verschieden Chlor, Brom oder eine $C_1$-$C_8$-Alkylamino- oder eine 1,2,4-Triazolylgruppe bedeuten, umgesetzt.

Vorzugsweise wurde das dansylierte Nukleosid mit Bis(2,2,2-Trifluoroethyl)H-phosphonat, Bis-(1,1,1,3,3,3-hexafluoro-2-propyl)4-phosphonat, Triethylphosphit, Triphenylphosphit umgesetzt oder mit $PCl_3$, Tri(dialkylamino)phosphinen oder Tris(1,2,4-Triazolyl) phosphit, vorzugsweise Tris(1,2,4-Triazolyl)phosphit, besonders bevorzugt mit $PCl_3$ nach Aktivierung mit Imidazol/ Triethylamin mit anschließender Hydrolyse zum H-Phosphonat.

Die Reaktion wurde in einem organischen Lösemittel wie Tetrahydrofuran oder Methylenchlorid, vorzugsweise Methylenchlorid, in Anwesenheit von 1-50 Moläquivalenten, vorzugsweise 10-50, insbesondere 30-50 Moläquivalente einer organischen Base wie $C_1$-$C_4$-Trialkylamin oder N-$C_1$-$C_4$-Alkylmorpholin, vorzugweise N-Methylmorpholin, durchgeführt. Das Molverhältnis von dansyliertem Nukleosid zum Phosphonilierungsreagenz betrug 1 zu 1 - 10, vorzugsweise 1 zu 2 - 8, insbesondere 1 zu 5.

Die so gewonnenen Verbindungen der Formel (VIIIa) oder (VIIIb) können anschließend für die Oligonukleotidsynthese eingesetzt werden. Hierbei sind die Zuckerreste der Nukleotide für die DNA-Synthese Desoxyribose, für die RNA-Synthese Ribose, aber es können auch Gemische aus Desoxyribose und Ribose für die Synthese eines Oligonukleotids bestehend aus regelmäßig oder unregelmäßig angeordneten Desoxyribose- und Ribosezuckerresten sein. Ferner kann das Oligonukleotid regelmäßig oder unregelmäßig aus Mononukleotiden der Formel (VIIIa) und (VIIIb) aufgebaut sein.

Die Oligonukleotidsynthese kann nach der H-Phosphonat-Methode, wie beispielsweise bei B.C. Froehler et al. (Froehler, B.C. (1986) Nucleic Acids Res. 14, 5399-5407, No. 13) beschrieben, durchgeführt werden, wobei die saure Abspaltung der 5'-Hydroxy-Schutzgruppe durch die basische Abspaltung der Dansylethoxycarbonylgruppe ersetzt wird.

Grundsätzlich kann die Synthese entweder in Lösung, beispielsweise analog nach C.B. Reese (1989), oder an der Festphase, beispielsweise analog nach B.C. Froehler (1986) erfolgen. Im allgemeinen ist die Festphasensynthese bevorzugt.

Hierbei wurde vorzugsweise die folgende Reaktionsfolge gewählt:

1. Umsetzung einer Verbindung der Formel (VIIIa) oder (VIIIb) mit dem Nukleosid-gebundenen polymeren Träger der Formel (VIIa) oder (VIIb) in Anwesenheit eines Säurechlorids, beispielsweise Pivaloylchlorid oder Adamantoylchlorid
2. Abspaltung der Dansylethoxycarbonylgruppe, beispielsweise mit DBU
3. Umsetzung der erhaltenen Verbindung mit einer Verbindung der Formel (VIIIa) oder (VIIIb)
4. Wiederholung der Schritte 2 und 3 bis zur gewünschten Kettenlänge des Oligonukleotids
5. Oxidation, zum Phosphat, Phosphoramidat oder zum Thiophosphat, beispielsweise mit Jod oder Schwefel oder Amin/$CCl_4$/Triphenylphosphin (Jäger et al. s.o.).

Die Verbindungen der Formel (Villa) oder (VIIIb) und (VIIa) und (VIIb) wurden vorzugsweise bei einer Temperatur von -20 °C bis +100 °C, insbesondere bei Raumtemperatur in Anwesenheit von beispielsweise Pivaloylchlorid als Säure umgesetzt. Die Oxidation erfolgte beispielsweise mit Jod in einem Lösemittelgemisch bestehend im allgemeinen aus Pyridin, N-Methylimidazol, Wasser, THF bei Raumtemperatur.

Die synthetisierte Oligonukleotide nach der beschriebenen H-Phosphonatmethode bestanden im allgemeinen aus 2 bis ca. 200, vorzugsweise 2 bis 110, insbesondere 2 bis 40 Mononukleotiden.

Die Vorteile der Oligonukleotidsynthese aus dansylierten Mononukleotiden nach der Phosphoramidit- oder nach der H-Phosphonat-Methode sind

a) leichte Nachweisbarkeit der Dansylgruppe wegen ihrer starken Fluoreszenz bei 550 nm,
b) Poly- bzw. Oligonukleotidsynthese bis in den Picomol-Bereich,
c) Entfernen der 5'-Hydroxy-Dansylschutzgruppe ohne Abspaltung anderer Hydroxy-Schutzgruppen an der Nukleotid-Base oder am Zuckerrest

d) Synthese von Oligoribonukleotiden und Oligodesoxyribonukleotiden, insbesondere von Oligoribonukleotiden,

e) Festphasensynthese von Oligonukleotiden in hohen Ausbeuten und in großer Kettenlänge.

Ein weiterer Vorteil der Verwendung der 5'-Hydroxy-Dansylschutzgruppe bei der RNA-Synthese ist, daß am Ende der Synthese die 2'-Hydroxygruppen des Riboserestes geschützt bleiben können. Die so modifizierten Oligoribonukleotide sind dadurch im allgemeinen vor Hydrolyse durch RNasen, aber auch vor möglichen Isomerierungsreaktionen geschützt und können daher über lange Zeiten stabil gelagert werden. Die 2'-Hydroxyschutzgruppe wird im allgemeinen dann erst kurz vor Gebrauch der RNA abgespalten.

Die Abspaltung vom Träger, sowie die Spaltung der Amino- und Hydroxy-Schutzgruppen an den synthetisierten Oligonukleotiden erfolgte nach allgemein bekannten Methoden z.B. wie bei M. Gait (Hrsg.): Oligonucleotide Synthesis, a practical approach; IRL Press; Oxford 1984 beschrieben.

Die folgenden Beispiele sollen die Erfindung näher erläutern. Als Abkürzungen wurden verwendet:

| | |
|---|---|
| Bz | für Benzoyl |
| Mthp | für Methoxytetrahydropyranyl |
| DansEOC | für Dansylethoxycarbonyl und |
| EE | für Essigsäureethylester |

Beispiel 1

Umsetzung von 2-Dansylethanol mit Trichlormethylchloroformiat

Unter Eiskühlung und Rühren pipettiert man 0,8 ml (1,32 g = 6,64 mmol) Trichlormethylchloroformiat in 10 ml absolutes $CH_3CN$. Danach tropft man unter Eiskühlung und Rühren durch eine Serumkappe mit einer Spritze 1 g (3,58 mmol) 2-Dansylethanol in 5 ml absolutem $CH_3CN$ gelöst zu. Man rührt im Eisbad 5 h weiter. Der ausgefallene farblose Feststoff wird abgesaugt, mit absolutem Tetrahydrofuran gewaschen und im Hochvakuum getrocknet. Man erhält 1,135 g (3,00 mmol = 84 %) eines farblosen Feststoffes vom Schmelzpunkt 154-55°.

Die Elementaranalyse ergibt:

| | gefunden | berechnet |
|---|---|---|
| Kohlenstoff | 47,90 % | 47,63 % |
| Wasserstoff | 4,64 % | 4,53 % |
| Stickstoff | 3,96 % | 3,70 % |

Beispiel 2

Umsetzung von 2'-O-(4-Methoxytetrahydropyranyl)-$N^6$-Benzoyl-Adenosin mit 2-Dansylethylchloroformiat-Hydrochlorid

2,43 g (5 mmol) 2'-O-Mthp-$N^6$-Bz-Adenosin werden 2 x mit je 30 ml absolutem Pyridin koevaporiert, dann in 40 ml absolutem Pyridin gelöst. Unter Eiskühlung und Rühren werden dann 2,46 g (6,5 mmol = 1,3 eq) 2-Dansylethylchloroformiat-Hydrochlorid in fester Form zugegeben. Man rührt 1 h im Eisbad, wobei nach ca. 0,5 h das Hydrochlorid gelöst ist. Danach stoppt man mit 0,5 ml (8,8 mmol) Glykol ab, rotiert ein, verdünnt mit 200 ml $CH_2Cl_2$, wäscht mit 200 ml gesättigter $NaHCO_3$-Lsg., extrahiert die wäßrige Phase 2 x mit je 100 ml $CH_2Cl_2$, trocknet die vereinigten organischen Phasen mit $Na_2SO_4$, filtriert ab und rotiert ein. Man koevaporiert 2 x mit je 100 ml Toluol und 2 x mit je 100 ml $CH_2Cl_2$. Man reinigt über eine $SiO_2$-Säule (100 g, 23 x 3,5 cm). Man eluiert mit 0,5 l $CH_2Cl_2$, 1 l $CH_2Cl_2$/MeOH 100:1 und 1,5 l $CH_2Cl_2$/MeOH 100:2 mit Flash-Chromatographie. Die einzelnen Produktfraktionen werden einrotiert und im Hochvakuum getrocknet. Man erhält 2,86 g (3,62 mmol = 72 %) 5'- und 0,47 g (0,43 mmol = 9 %) 3',5'-di-substituiertes Produkt jeweils als gelbe, stark fluoreszierende Schäume. Für die Elementar-Analyse werden 100 mg des disubstituierten Produktes nochmals über 1 $SiO_2$-Platte (40 x 20 cm) mit $CH_2Cl_2$/MeOH 100:2 gereinigt.

Analysendaten für 2'-O-(4-Methoxytetrahydropyranyl)-5'-O-dansyl-ethoxycarbonyl-$N^6$-benzoyl-adenosin

a) Dünnschichtchromatographie

Die Dünnschichtchromatographie wurde auf Schleicher und Schüll Kieselgel F 1500/LS 254 in $CH_2Cl_2$/MeOH (95:5) durchgeführt und ein $R_f$-Wert von 0,48 berechnet.

b) UV-Spektroskopie in Methanol:

| | | | |
|---|---|---|---|
| λmax | 257 | 272 | 339 |
| $\log_{\epsilon max}$ | 4,42 | 4,38 | 3,65 |

c) Elementaranalyse

| | gefunden | berechnet mit 1 Mol $H_2O$ |
|---|---|---|
| Kohlenstoff | 56,58 % | 56,43 % |
| Wasserstoff | 5,46 % | 5,48 % |
| Stickstoff | 10,28 % | 10,39 % |

d) NMR-Spektroskopie

in $CDCl_3$ bei 250 MHz
9,03 s breit (1) NH, 8,81 s (1) H-8, 8,61 d (2) Dansyl-H-2, 8,35-8,30 m (2) Dansyl-H-4, Dansyl-H-8, 8,23 s (1) H-2, 8,03 d (2) 2H von o-Bz, 7,65-7,50 m (5) 3H von Bz, Dansyl-H-3, Dansyl-H-7, 7,20 d (1) Dansyl-H-6, 6,20 d (1) H-1', 5,13 t (1) H-2', 4,52-4,32 m (6) $CH_2OCO$, H-3', H-4', H-5', H-5'', 3,72 t (2) $SO_2CH_2$, 3,77-3,43 m (4) $CH_2OCH_2$ (Mthp), um 2,90 s breit (1) 3'-OH, 2,89 s (6) $NMe_2$, 2,87 s (3) $OCH_3$, 1,95-1,55 m (4) $CH_2CCH_2$ (Mthp)

Analysendaten für 2'-O-(4-Methoxytetrahydropyranyl)-3',5'-O-bis-dansylethoxycarbonyl-$N^6$-benzoyl-adenosin

a) Dünnschichtchromatographie

Die Dünnschichtchromatographie wurde auf Kieselgel F 1500/LS 254 (Schleicher & Schüll) in $CH_2Cl_2$/MeOH (100:2) durchgeführt und ein $R_f$-Wert von 0,35 berechnet.

b) UV-Spektroskopie in Methanol:

| | | | |
|---|---|---|---|
| λmax | 254 | 278 | 343 |
| $\log_{\epsilon max}$ | 4,58 | 4,37 | 3,91 |

c) Elementaranalyse

| | gefunden | berechnet |
|---|---|---|
| Kohlenstoff | 57,87 % | 58,07 % |
| Wasserstoff | 5,46 % | 5,24 % |
| Stickstoff | 8,67 % | 8,94 % |

d) NMR-Spektroskopie

in $CDCl_3$ bei 250 MHz
9,05 s breit (1) NH, 8,83 s (1) H-8, 8,69-8,60 2d (2) 2 x Dansyl-H-2, 8,35-8,29 m (4) 2 x Dansyl-H-4, 2 x Dansyl-H-8, 8,22 s (1) H-2, 8,03 d (2) 2H von o-Bz, 7,68-7,50 m (7) 3H von Bz, 2 x Dansyl-H-3, 2 x Dansyl-H-7, 7,24-7,18 2 zu t überlagerte d (2) 2 x Dansyl-H-6, 6,13 d (1) H-1', 5,32 t (1) H-2', 5,13-5,11 m (1) H-3', 4,60-4,22 m (7) 2 x $CH_2OCO$, H-4', H-5', H-5'', 3,77-3,26 m (8) 2 x $SO_2CH_2$, $CH_2OCH_2$ (Mthp), 2,89 s (6) $NMe_2$, 2,88 s (6) $MMe_2$, 2,63 s (3) $OCH_3$, 1,78-1,20 m (4) $CH_2CCH_2$ (Mthp)

Beispiel 3

Umsetzung von 2'-O-(4-Methoxytetrahydropyranyl)-5'-O-dansylethoxycarbonyl-$N^6$-benzoyl-adenosin mit Phosphorigsäurechlorid-2-(4-nitrophenyl)-ethylester-N,N-diisopropylamid

1 g (1,264 mmol) 2'-O-Mthp-5'-O-DansEOC-$N^6$-Bz-Adenosin werden in 6 ml absolutem $CH_2Cl_2$ gelöst, dann werden 0,86 ml (0,65 g = 5,03 mmol = 4 eq) Hünigs Base und 0,84 g (2,528 mmol = 2 eq) Phosphorigsäurechlorid 2-(4-nitrophenyl)-ethylester-N,N-diisopropylamid zugegeben. Unter einer Stickstoffatmosphäre, abgedunkelt mit einer Alufolie, wird der Ansatz bei Raumtemperatur gerührt. Nach 1 1/4 h werden nochmals 0,42 g (1,264 mmol = 1 eq) Phosphitylierungreagenz zugegeben. Nach insgesamt 2,5 h Rühren bei Raumtemperatur wird mit 75 ml $CH_2Cl_2$ verdünnt, mit 75 ml gesättigter $NaHCO_3$-Lsg. gewaschen, die wäßrige Phase 4 x mit je 50 ml $CH_2Cl_2$ rückextrahiert, die vereinigten organischen Phasen über $Na_2SO_4$ getrocknet, abfiltriert und einrotiert. Man reinigt über eine $SiO_2$-Säule (30 g, 12 x 3 cm) mit Flash-Chromatographie: Man eluiert mit 250 ml $CH_2Cl_2$, 100 ml $CH_2Cl_2$/EE 100:1, 100 ml $CH_2Cl_2$/EE 100:2, 100 ml $CH_2Cl_2$/EE 100:3, 100 ml $CH_2Cl_2$/EE 100:5, 100 ml $CH_2Cl_2$/EE 100:7, 100 ml $CH_2Cl_2$/EE 9:1, 350 ml $CH_2Cl_2$/EE 4:1 (Reagenz), 100 ml $CH_2Cl_2$/EE 2:1 (Produkt), 100 ml $CH_2Cl_2$/EE 1:1 (Produkt), 100 ml $CH_2Cl_2$/EE 1:2 (Produkt) und 100 ml EE (Produkt).

Die Produktfraktionen werden einrotiert und im Hochvakuum getrocknet.
Man erhält 0,955 g (0,878 mmol = 70 %) eines gelben, fluoreszierenden Schaumes.

Analysendaten

a) Dünnschichtchromatographie

Die Dünnschichtchromatographie wurde auf Schleicher & Schüll F 1500/LS 254 Kieselgel in Toluol/EE (1:6) durchgeführt und ein $R_f$-Wert von 0,50 berechnet.

b) UV-Spektroskopie in Methanol

| | | | |
|---|---|---|---|
| $\lambda max$ | 261 | 330 | 339 |
| $\log_{\varepsilon max}$ | 4,54 | 3,55 | 3,51 |

c) Elementaranalyse

| | gefunden | berechnet |
|---|---|---|
| Kohlenstoff | 57,08 % | 57,45 % |
| Wasserstoff | 5,87 % | 5,84 % |
| Stickstoff | 10,29 % | 10,31 % |

d) NMR-Spektroskopie

1. $^{31}$P-NMR in $CDCl_3$ bei 161,70 MHz
151,34 ppm s (31 %)
149,47 ppm s (69 %)
2. $^{1}$H-NMR in $CDCl_3$ bei 250 MHz
9,07 s breit (1) NH, 8,83 u. 8,82 2s (1) H-8, 8,61 d (1) Dansyl-H-2, 8,35-8,29 m (2) Dansyl-H-4, Dansyl-H-8, 8,24 u. 8,22 2s (1) H-2, 8,18 -8,13 2d (2) 2H o zu Phenyl-$NO_2$, 8,03 d (2) 2H von o-Bz, 7,64-7,49 m (5) 3H von Bz, Dansyl-H-3, Dansyl-H-7, 7,45-7,38 2d (2) 2H m zu Phenyl-$NO_2$, 7,19 d (1) Dansyl-H-6, 6,23 u. 6,15 2d (1) H-1', 5,20 u. 5,11 2t (1) H-2', 4,48 t (2) $CH_2OCO$, 4,44-4,18 m (4) H-3', H-4', H-5', H-5'', 4,10-3,88 m (2) $CH_2OP$, 3,84-3,25 m (6) 2 x CH
(i-Pr). $CH_2OCH_2$ (Mthp), 3,71 t (2) $SO_2CH_2$, 3,06 t (2) $CH_2$-Phenyl-$NO_2$, 2,88 s (6) $NMe_2$, 2,66 u. 2,61 2s (3) $OCH_3$, 2,00-1,45 m (4) $CH_2CCH_2$ (Mthp), 1,26-1,12 überlagerte d (12) 2 x $C(CH_3)_2$ (i-Pr)

Beispiel 4

Automatische Oligoribonukleotid-Synthese mit 2'-O-(4-Methoxy-tetrahydropyranyl)-5'-O-dansylethoxycarbonylphosphitamidit. Herstellung des Decanucleotids $(rAp)_9T$.

Die Synthesen wurden mit einem 380 B DNA-Synthesizer (Applied Biosystems) durchgeführt.

Verwendete Säule: ABI-Standardsäule

Verwendetes Trägermaterial: LCAMA-CPG-Träger, der mit dem Nucleosid über 3'-Hydroxygruppe verbunden ist.

(Literatur: K.P. Stengele, W. Pfleiderer Nucleic Acids Res. Symp. Ser. 21, 101 (1989); K.P. Stengele, W. Pfleiderer Tetrahedron Lett. 31, 2549 (1990)).

Beladung mit Thymidin, das über die 3'-Hydroxy-Gruppe an den Träger gebunden ist; 19 µmol/g
Ansatzgröße: ca. 0,6 µmol (Bestimmung durch Tritylabspaltung).

Synthesezyklus:

1. Kondensation mit 0,5 M Tetrazol und 0,1 M 2'-O-(4-Methoxy-tetrahydropyranyl)-5'-O-dansylethoxycarbonyl-$N^6$-benzoyl-adenosin-3'-O-phosphitamid in absolutem Acetonitril gemäß folgender Pulsfolge:

| | |
|---|---|
| Tetrazol | 8 sec. |
| Phosphitamid + Tetrazol | 4 sec. |
| Tetrazol | 3 sec. |
| Phosphitamid + Tetrazol | 3 sec. |
| Tetrazol | 3 sec. |
| Warteschritt | 60 sec. |
| Phosphitamid + Tetrazol | 3 sec. |
| Tetrazol | 3 sec. |
| Warteschritt | 700 sec. |

2. Capping von nicht-umgesetzten Nukleotid mit Acetanhydrid/Lutidin/THF (1:1:3) und 6,5 % Dimethyl aminopyridin (DMAP) in THF

| | |
|---|---|
| Durchfluß | 20 sec. |
| Warteschritt | 30 sec. |

3. Oxidation mit $I_2$-Lösung (1.269 g $I_2$/20 ml $H_2O$/10 ml Pyridin/100 ml THF)

| | |
|---|---|
| Durchfluß | 30 sec. |
| Warteschritt | 30 sec. |

4. Dansylethoxycarbonyl-Abspaltung mit 0,1 M DBU in Acetonitril in 2 x 30 sec. und 8 x 10 sec. gepulsten Flüssen mit dazwischengeschalteten 1 sec. "reverse flushes".

Die Eluate aus dem 4. Schritt wurden gesammelt und die Kondensationsausbeuten anhand des gebildeten 5-Dimethylamino-naphtyl-1-vinylsulfons mittels Fluoreszenzspektroskopie (Anregung: 368 nm; Emission: 526 nm) bestimmt.

Die durchschnittliche stufenweise Ausbeute betrug ca. 98 %.

Zwischen den einzelnen Schritten 1-4 erfolgten die üblichen Waschschritte mit Acetonitril sowie die "block- bzw. reverse flushes".

Beispiel 5

Synthese von 5'-O-Dansylethoxycarbonyl-geschützten H-Phosphonaten

10,75 Äquivalente Imidazol werden in 5 ml absolutem Methylenchlorid gelöst, dann mit Eis/Kochsalz gekühlt und anschließend die gekühlte Lösung mit 3,5 Äquivalenten $PCl_3$ und 11.25 Äquivalenten Triethylamin versetzt. Man rührt 15 Minuten unter Kühlung und gibt dann 0,25 mmol (1 Äquivalent) 2'-O-(4-Methoxytetrahydropyranyl)-5'-O-dansyl-ethoxycarbonyl-$N^6$-benzoyladenosin oder 0,25 mmol 2'-O-(4-Methoxytetrahydropyranyl)-5'-O-dansylethoxycarbonyl-$N^6$-para-nitrophenylethyloxycarbonyladenosin (1 x mit Acetonitril koevaporiert) in 5 ml absolutem Methylenchlorid innerhalb von 10 min tropfenweise unter Rühren zu.

Danach wird das Eisbad entfernt und weitere 15 Minuten bei Raumtemperatur gerührt. Anschließend wird die Reaktionslösung mit 10 ml 1 M Triethylammonium-bikarbonat ausgeschüttelt. Man trennt die Phasen, extrahiert die wäßrige Phase mit 10 ml $CH_2Cl_2$ trocknet die vereinigten organischen Phasen über $Na_2SO_4$, filtriert ab und rotiert zu einem gelben, fluoreszierenden Schaum ein. Man reinigt über eine kurze Kieselgel-Säule (Flash-Chromatographi) mit einem $CH_2Cl_2$/MeOH-Gradienten.

Beispiel 6

Automatische Oligoribonukleotid-Synthese mit 2'-O-(4-Methoxy-tetrahydropyranyl)-5'-O-dansylethoxycarbonylribonucleosid-3'-O-H-phosphonaten. Herstellung von $(rAp)_gT$.

Die Synthesen wurden mit einem 380 B DNA-Synthesizer (Applied Biosystems) durchgeführt.
Verwendete Säule: ABI-Standardsäule
Verwendetes Trägermaterial: LCAMA-CPG-Träger (Literatur: K.P. Stengele, W. Pfleiderer Nucleic Acids Res. Symp. Ser. 21, 101 (1989); K.P. Stengele, W. Pfleiderer Tetrahedron Lett. 31, 2549 (1990)).
Beladung mit Thymidin, das über die 3'-Hydroxy-Gruppe an den Träger gebunden ist, 19 µmol/g
Ansatzgröße: ca. 0,6 µmol (Bestimmung durch Tritylabspaltung).

Synthesezyklus:

1. Waschen mit absolutem Pyridin/Acetonitril (1:1)
2. Umsetzung mit 2'-O-(4-Methoxytetrahydropyranyl)-5'-O-dansylethoxycarbonyl-H-phosphonat (10 mM) und Pivaloylchlorid (50 mM) in absolutem Pyridin/Acetonitril (1:1)
3. Waschen mit absolutem Acetonitril (45 Sekunden)
4. Dansylethoxycarbonyl-Abspaltung mit 0,1 M DBU in Acetonitril (2 Minuten)
5. Wiederholung der Schritte 1 bis 4 bis gewünschte Kettenlänge erreicht.
6. Dansylethoxycarbonyl-Abspaltung mit 0,1 M DBU in Acetonitril (2 Minuten) und Sammeln der Eluate

7. Oxidation mit $J_2$ (0,1 M) in Pyridin/N-Methylimidazol/Wasser/THF (5/1/5/90) (2,5 Minuten) oder mit $J_2$ - (0,1 M) in Triethylamin/Wasser/THF (5/5/90) (2,5 Minuten)

Die Oxidation zum Thiophosphat oder Phosphoramidat wurde auch wie in Uhlmann & Peyman (1990) beschrieben durchgeführt.

Die Eluate aus dem 6. Schritt werden gesammelt und die Kondensationsausbeuten anhand des gebildeten 5-Dimethylamino-naphthyl-1-vinylsulfons mittels Fluoreszenzspektroskopie (Anregung: 368 nm; Emission: 526 nm) bestimmt.

Die durchschnittliche stufenweise Ausbeute beträgt ca. 98 %.

## Patentansprüche

**Patentansprüche für folgende Vertragsstaaten : AT, BE, CH, DE, DK, FR GB, GR, IT, LI, LU, NL, SE**

**1.** Verfahren zur Herstellung der Verbindung der Formel (I),

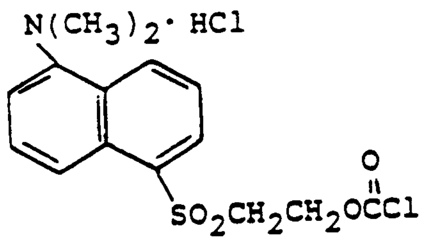

(I),

dadurch gekennzeichnet, daß die Verbindung der Formel (II),

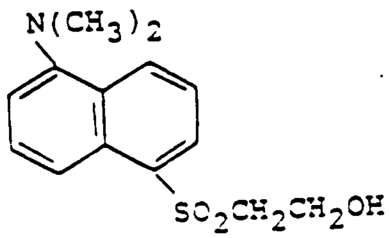

(II),

mit einem Chlorcarbonyl-Donor umgesetzt wird.

**2.** Verbindung der Formel (IIIa) oder (IIIb)

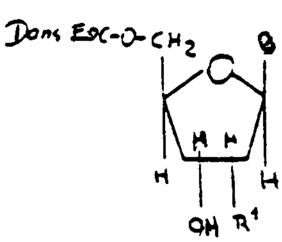

(IIIa)

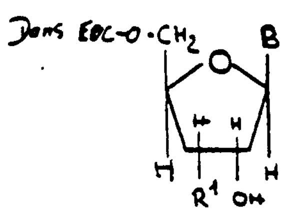

(IIIb)

worin bedeuten:
DansEOC eine Gruppe der Formel 1

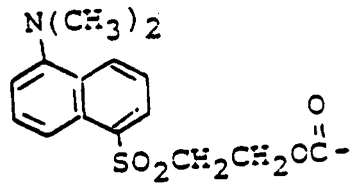

$R^1$ Wasserstoff oder unabhängig voneinander

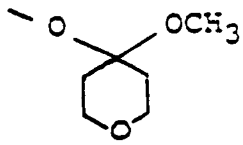

eine Gruppe der Formel

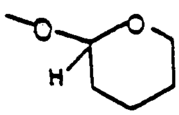 ,

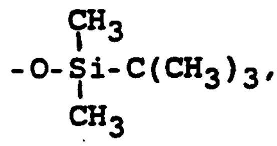

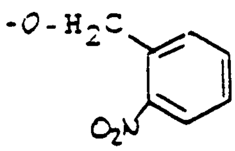 ,

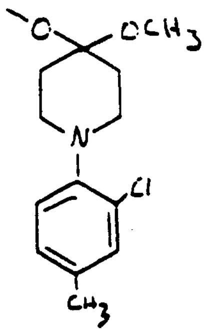

oder

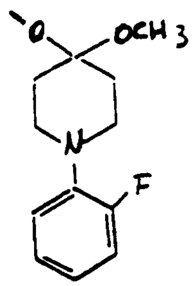

und

B bedeutet

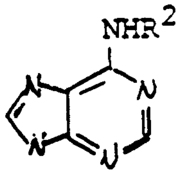 ,

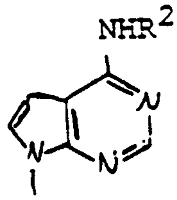 ,

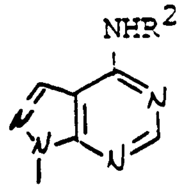 ,

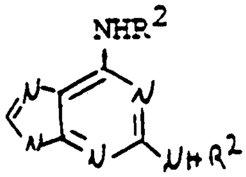

oder

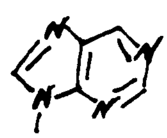

mit $R^2$ jeweils unabhängig voneinander eine Gruppe der Formel

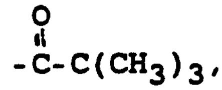, 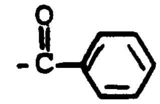

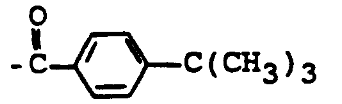 oder 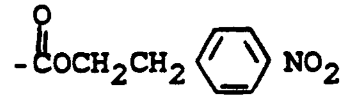

oder

B bedeutet

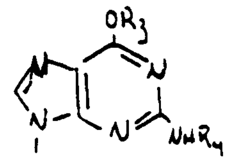, 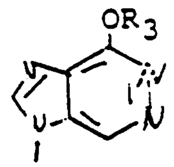 oder 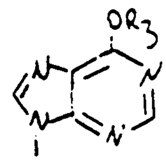

mit $R^3$ jeweils unabhängig voneinander Wasserstoff oder

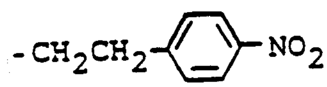

und

$R^4$ jeweils unabhängig voneinander eine Gruppe der Formel

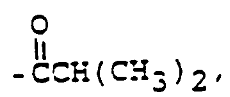, 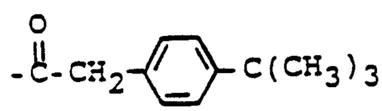 oder

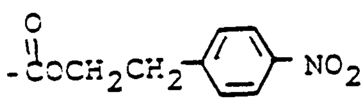

oder

B bedeutet

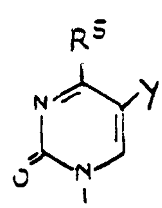 oder 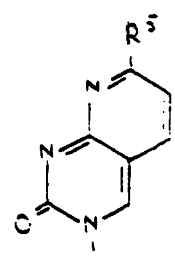

Y = H, $CH_3$ (oder: n-Alkyl $C_1$-$C_4$) mit $R_5$ jeweils unabhängig voneinander -OH,

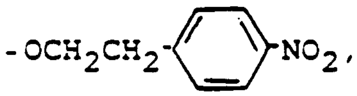,

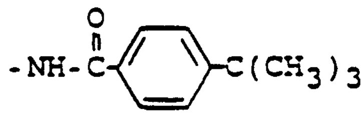 oder 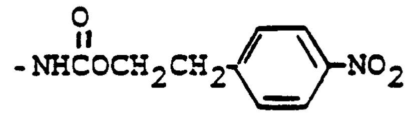

3. Verfahren zur Herstellung einer Verbindung der Formel (IIIa) oder (IIIb) durch Umsetzung der Verbindung der Formel (I) mit einer entsprechenden Verbindung der Formel (IVa) oder (IVb)

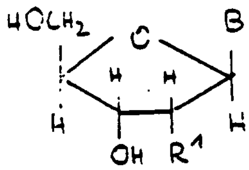 (IVa) 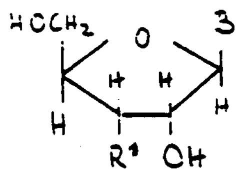 (IVb)

worin $R^1$ und B die obige Bedeutung haben, in Gegenwart einer Base.

4. Verbindung der Formel (Va) oder (Vb)

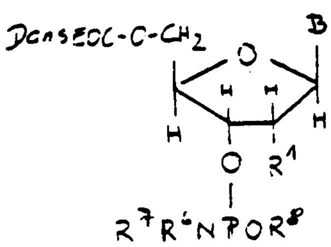 (Va) 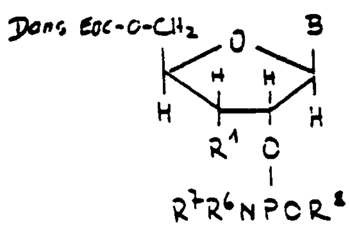 (Vb)

worin
DansEOC, $R^1$ und B die obengenannte Bedeutung haben und
$R^6$, $R^7$ gleich oder unabhängig verschieden eine $C_1$-$C_8$-Alkyl-, vorzugsweise eine Isopropyl- oder $C_5$-$C_{12}$-Cycloalkyl-Gruppe, vorzugsweise bis $C_8$, eine Benzyl oder Phenyl-Gruppe oder zusammen mit dem Stickstoffatom, an das sie gebunden sind, einen gesättigten oder ungesättigten heterocyclischen Ring, der gegebenenfalls weitere Heteroatome und Substituenten enthalten kann und
$R^8$ eine Gruppe der Formel

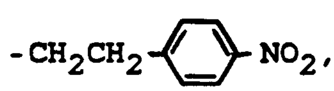, 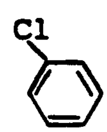

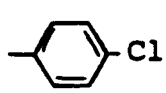

oder $CH_3$ oder eine Benzylgruppe, welche nicht substituiert oder einfach oder mehrfach ringsubstituiert, vorzugsweise nicht substituiert ist, wobei der oder die Substituenten unabhängig voneinander ein Halogen, eine $C_1$-$C_4$-Alkyl-, Nitro-, Methoxy- oder Carboxylgruppe ist.

**5.** Verfahren zur Herstellung einer Verbindung der Formel (Va) oder (Vb) durch Umsetzung der Verbindung der Formel (IIIa) oder (IIIb) mit einer Verbindung der Formel (VI)

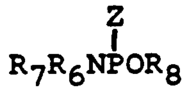

(VI),

worin $R_6$, $R_7$ und $R_8$ die obige Bedeutung haben und Z Chlor oder Brom oder ein Rest der Formel $-NR_9R_{10}$, wobei $R_9$, $R_{10}$ gleich oder unabhängig verschieden eine $C_1$-$C_8$-Alkyl-, vorzugsweise eine Isopropyl- oder $C_5$-$C_{12}$-Cycloalkyl-Gruppe, vorzugsweise bis $C_8$, eine Benzyl- oder eine Phenyl-Gruppe, vorzugsweise Chlor ist, in Gegenwart einer Base.

**6.** Verfahren zur Herstellung von Oligonukleotiden aus Verbindungen der Formel (Va) und/oder (Vb), dadurch gekennzeichnet, daß man eine Verbindung der Formel (Va) oder (Vb)

1. mit einer Verbindung der Formel (VIIa) oder (VIIb),

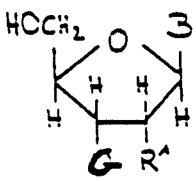

(VIIa) oder

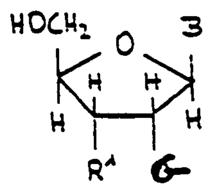

(VIIb)

worin B und $R^1$ die obengenannte Bedeutung haben, und G gleichbedeutend mit $R^1$ oder ein polymerer Träger ist, der über die 2'-Hydroxy- oder 3'-Hydroxy-Gruppe der Verbindung der Formel (VIIa) oder (VIIb) gebunden ist, umsetzt,
2. die erhaltene Verbindung oxidiert,
3. die Dansylethoxycarbonyl-Gruppe abspaltet,
4. die erhaltene Verbindung mit einer Verbindung der Formel (Va) oder (Vb) umsetzt und
5. die Reaktionsschritte 2-4 bis zur gewünschten Kettenlänge wiederholt.

**7.** Verbindung der Formel (VIIIa) oder (VIIIb)

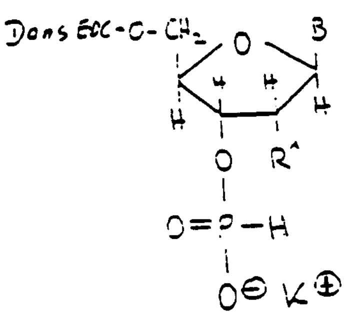

(VIIIa)

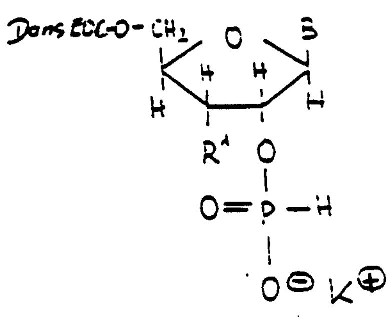

(VIIIb)

worin DansEOC, $R^1$ und B die obengenannte Bedeutung haben und $K^{(+)}$ ein Kation, insbesondere $[HN-(C_2H_5)_3]^{(+)}$ ist.

**8.** Verfahren zur Herstellung einer Verbindung der Formel (VIIIa) oder (VIIIb) durch Umsetzung einer Verbindung der Formel (IIIa) oder (IIIb) mit einer Verbindung der Formel (IX)

$PR_{14}R_{15}R_{16}$ (IX)

worin $R_{14}$, $R_{15}$, $R_{16}$ gleich oder unabhängig verschieden Wasserstoff oder eine $C_1$-$C_8$-Alkyl-, $C_1$-$C_8$-Fluoralkyl- oder Aryl-Gruppe, vorzugsweise eine 2,2,2-Trifluorethyl-, 1,1,1,3,3,3-Hexafluoro-2-propyl-, Ethyl- oder Phenyl-Gruppe, in Gegenwart einer Base.

**9.** Verfahren zur Herstellung einer Verbindung der Formel (VIIIa) oder (VIIIb) durch Umsetzung einer Verbindung der Formel (IIIa) oder (IIIb) mit einer Verbindung der Formel (X)

$PR_{17}R_{18}R_{19}$ (X)

worin $R_{17}R_{18}R_{19}$ gleich oder unabhängig verschieden Chlor, Brom oder eine $C_1$-$C_8$-Alkylamino- oder eine 1,2,4-Triazolylgruppe, vorzugsweise eine 1,2,4-Triazoylgruppe, in Gegenwart einer Base mit anschließender Hydrolyse.

**10.** Verfahren zur Herstellung von Oligonukleotiden aus Verbindungen der Formel (VIIIa) und/oder (VIIIb), dadurch gekennzeichnet, daß man eine Verbindung der Formel (VIIIa) oder (VIIIb)
1. mit einer Verbindung der Formel (VIIa) oder (VIIb) umsetzt,
2. die Dansylethoxycarbonyl-Gruppe abspaltet,
3. die erhaltene Verbindung mit einer Verbindung der Formel (VIIIa) oder (VIIIb) umsetzt,
4. die Reaktionsschritte 2 und 3 bis zur gewünschten Kettenlänge wiederholt und
5. das erhaltene Oligonukleotid oxidiert.

**Patentansprüche für folgenden Vertragsstaat : ES**

**1.** Verfahren zur Herstellung der Verbindung der Formel (I),

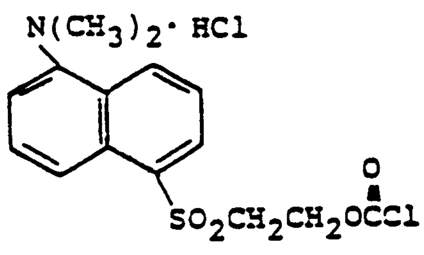

(I),

dadurch gekennzeichnet, daß die Verbindung der Formel (II),

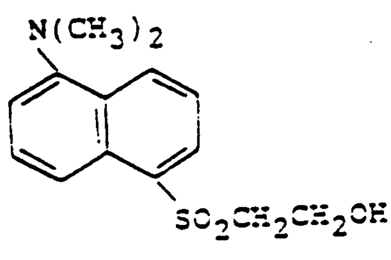

(II),

mit einem Chlorcarbonyl-Donor umgesetzt wird.

**2.** Verfahren zur Herstellung einer Verbindung der Formel (IIIa) oder (IIIb)

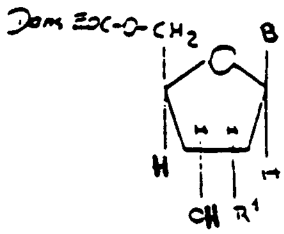 (IIIa) 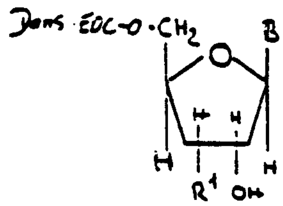 (IIIb)

worin bedeuten:
DansEOC eine Gruppe der Formel

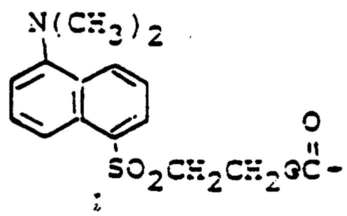

$R^1$ Wasserstoff oder unabhängig voneinande

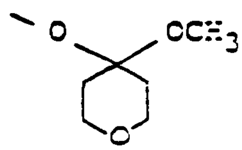

eine Gruppe der Formel

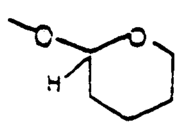 , 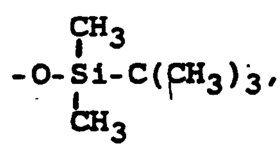

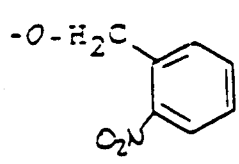 , 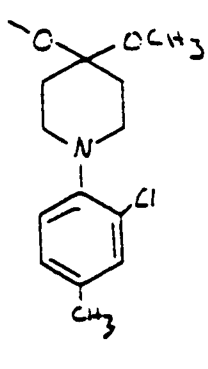 oder 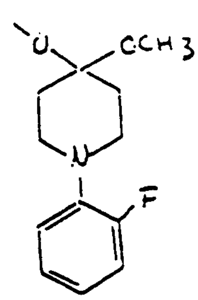

und
B bedeutet

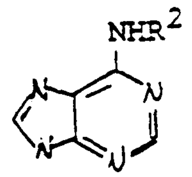,
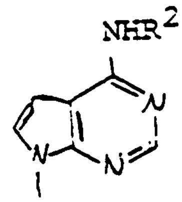,
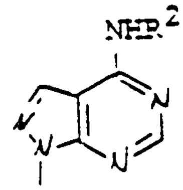,

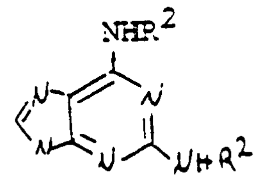
oder
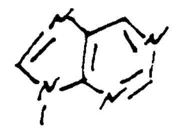.

mit $R^2$ jeweils unabhängig voneinander eine Gruppe der Formel

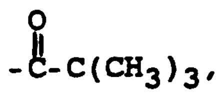,
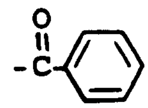

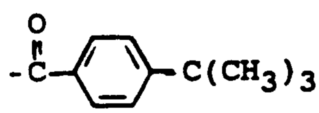
oder
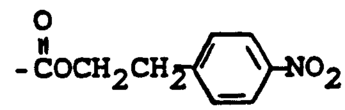

oder

B bedeutet

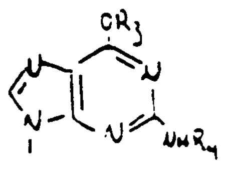,
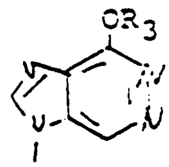
oder
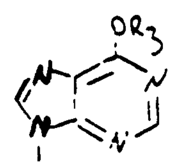

mit $R^3$ jeweils unabhängig voneinander Wasserstoff oder

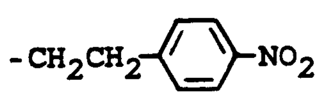

und

$R^4$ jeweils unabhängig voneinander eine Gruppe der Formel

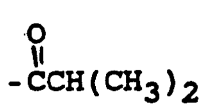,
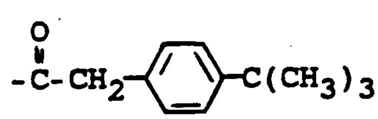
oder

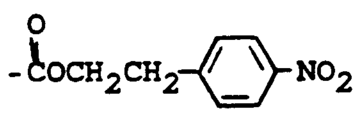

oder

B bedeutet

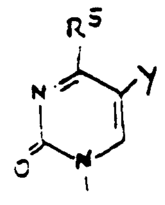

oder

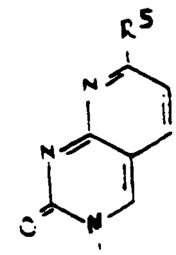

Y = H, $CH_3$ (oder: n-Alkyl $C_1$-$C_4$)
mit $R_5$ jeweils unabhängig voneinander -OH,

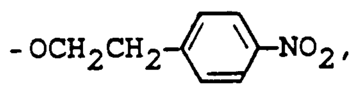

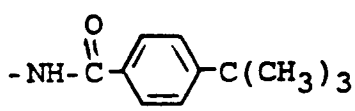

oder

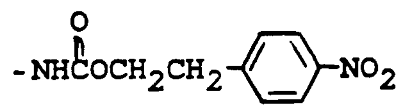

durch Umsetzung der Verbindung der Formel (I) mit einer entsprechenden Verbindung der Formel (IVa) oder (IVb)

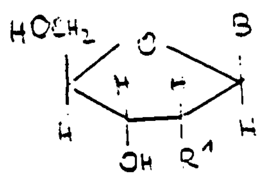

(IVa)

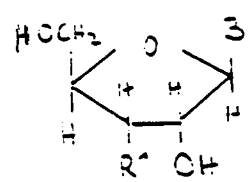

(IVb)

worin $R^1$ und B die obige Bedeutung haben, in Gegenwart einer Base.

**3.** Verfahren zur Herstellung einer Verbindung der Formel (Va) oder (Vb)

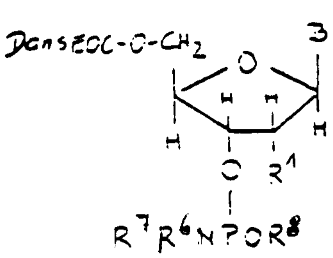

(Va)

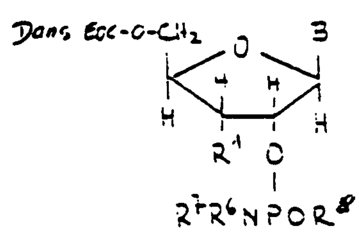

(Vb)

worin
DansEOC, $R^1$ und B die obengenannte Bedeutung haben und
$R^6$, $R^7$ gleich oder unabhängig verschieden eine $C_1$-$C_8$-Alkyl-, vorzugsweise eine Isopropyl- oder $C_5$-$C_{12}$-Cycloalkyl-Gruppe, vorzugsweise bis $C_8$, eine Benzyl oder Phenyl-Gruppe oder zusammen mit dem Stickstoffatom, an das sie gebunden sind, einen gesättigten oder ungesättigten heterocyclischen Ring, der gegebenenfalls weitere Heteroatome und Substituenten enthalten kann und

$R^8$ eine Gruppe der Formel

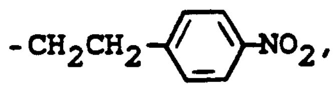, 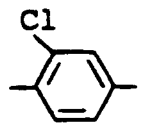

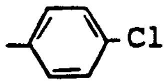

oder $CH_3$
oder eine Benzylgruppe, welche nicht substituiert oder einfach oder mehrfach ringsubstituiert, vorzugsweise nicht substituiert ist, wobei der oder die Substituenten unabhängig voneinander ein Halogen, eine $C_1$-$C_4$-Alkyl-, Nitro-, Methoxy- oder Carboxylgruppe ist, durch Umsetzung der Verbindung der Formel (IIIa) oder (IIIb) mit einer Verbindung der Formel (VI)

$$R_7R_6N\overset{Z}{P}OR_8 \qquad (VI),$$

worin $R_6$, $R_7$ und $R_8$ die obige Bedeutung haben und Z Chlor oder Brom oder ein Rest der Formel $-NR_9R_{10}$, wobei $R_9$, $R_{10}$ gleich oder unabhängig verschieden eine $C_1$-$C_8$-Alkyl-, vorzugsweise eine Isopropyl- oder $C_5$-$C_{12}$-Cycloalkyl-Gruppe, vorzugsweise bis $C_8$, eine Benzyl- oder eine Phenyl-Gruppe, vorzugsweise Chlor ist, in Gegenwart einer Base.

**4.** Verfahren zur Herstellung von Oligonukleotiden aus Verbindungen der Formel (Va) und/oder (Vb), dadurch gekennzeichnet, daß man eine Verbindung der Formel (Va) oder (Vb)
1. mit einer Verbindung der Formel (VIIa) oder (VIIb),

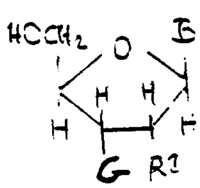 **(VIIa) oder** 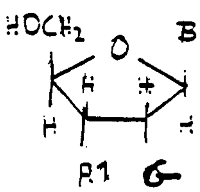 **(VIIb)**

worin B und $R^1$ die obengenannte Bedeutung haben, und G gleichbedeutend mit $R^1$ oder ein polymerer Träger ist, der über die 2'-Hydroxy- oder 3'-Hydroxy-Gruppe der Verbindung der Formel (VIIa) oder (VIIb) gebunden ist, umsetzt,
2. die erhaltene Verbindung oxidiert,
3. die Dansylethoxycarbonyl-Gruppe abspaltet,
4. die erhaltene Verbindung mit einer Verbindung der Formel (Va) oder (Vb) umsetzt und
5. die Reaktionsschritte 2-4 bis zur gewünschten Kettenlänge wiederholt.

**5.** Verfahren zur Herstellung einer Verbindung der Formel (VIIIa) oder (VIIIb)

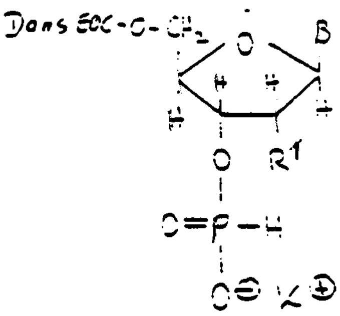

(VIIIa)

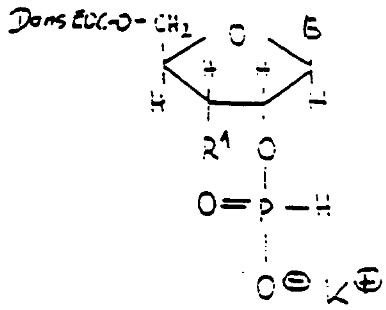

(VIIIb)

worin DansEOC, $R^1$ und B die obengenannte Bedeutung haben und $K^{(+)}$ ein Kation, insbesondere $[HN(C_2H_5)_3]^{(+)}$ ist, durch Umsetzung einer Verbindung der Formel (IIIa) oder (IIIb) mit einer Verbindung der Formel (IX)

$PR_{14}R_{15}R_{16}$ (IX)

worin $R_{14}$, $R_{15}$, $R_{16}$ gleich oder unabhängig verschieden Wasserstoff oder eine $C_1$-$C_8$-Alkyl-, $C_1$-$C_8$-Fluoralkyl- oder Aryl-Gruppe, vorzugsweise eine 2,2,2-Trifluorethyl-, 1,1,1,3,3,3-Hexafluoro-2-propyl-, Ethyl- oder Phenyl-Gruppe, in Gegenwart einer Base.

**6.** Verfahren zur Herstellung einer Verbindung der Formel (VIIIa) oder (VIIIb)

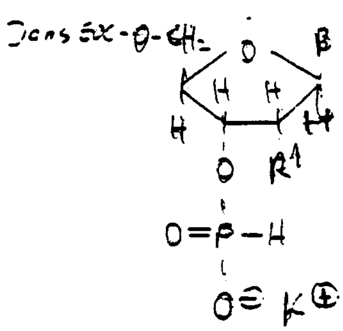

(VIIIa)

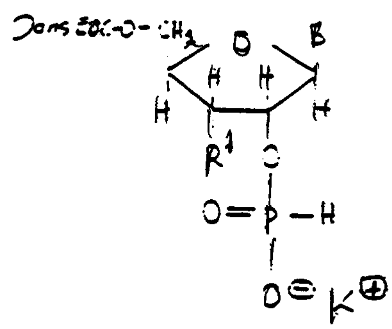

(VIIIb)

worin DansEOC, $R^1$ und B die obengenannte Bedeutung haben und $K^{(+)}$ ein Kation, insbesondere →-$[HN(C_2H_5)_3$→$]^{(+)}$ ist, durch Umsetzung einer Verbindung der Formel (IIIa) oder (IIIb) mit einer Verbindung der Formel (X)

$PR_{17}R_{18}R_{19}$ (X)

worin $R_{17}R_{18}R_{19}$ gleich oder unabhängig verschieden Chlor, Brom oder eine $C_1$-$C_8$-Alkylamino- oder eine 1,2,4-Triazolylgruppe, vorzugsweise eine 1,2,4-Triazoylgruppe, in Gegenwart einer Base mit anschließender Hydrolyse.

**7.** Verwendung einer Verbindung der Formel IIIa, IIIb, Va, Vb, VIIIa oder VIIIb in der Oligonukleotidsynthese.

**8.** Verfahren zur Herstellung von Oligonukleotiden und Dansylethoxycarbonyl-geschützter Oligonukleotide, hergestellt aus mindestens einer Verbindung der Formel (Va und/oder (Vb).

## Claims

**Claims for the following Contracting States : AT, BE, CH, DE, DK, FR, GB, GR, IT, LI, LU, NL, SE**

1. A process for the preparation of the compound of the formula (I),

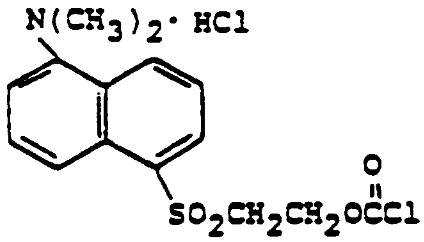

(I),

which comprises reacting the compound of the formula (II),

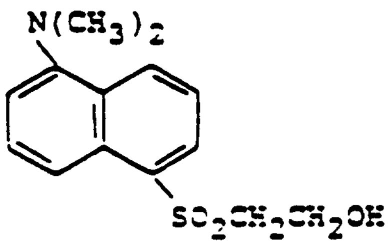

(II),

with a chlorocarbonyl donor.

2. A compound of the formula (IIIa) or (IIIb)

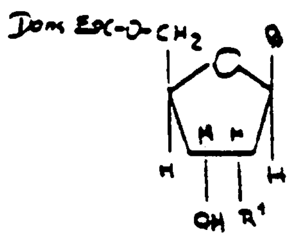

(IIIa)

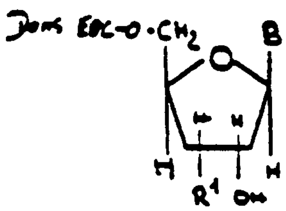

(IIIb)

in which DansEOC is a group of the formula

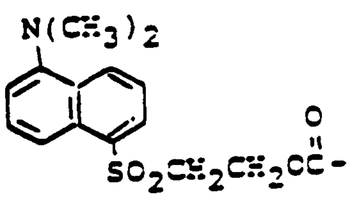

$R^1$ is hydrogen or, independently of one another, a group of the formula

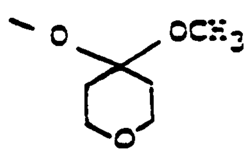

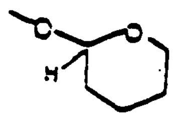
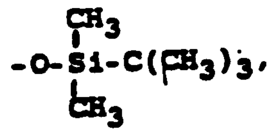
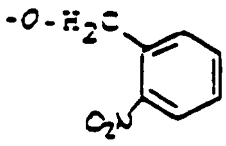
,
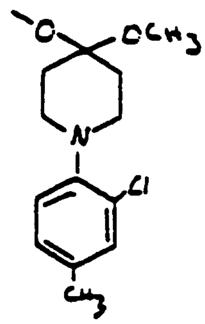
or
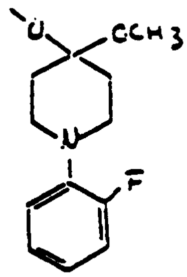

and

B is

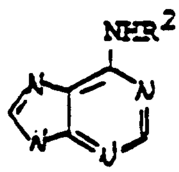
,
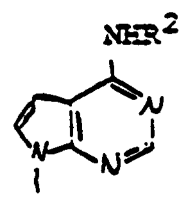
,
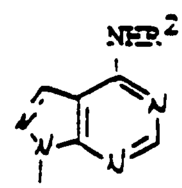
,
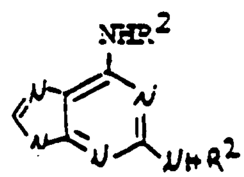
or
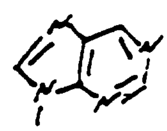

with $R^2$ being, in each case independently of one another, a group of the formula

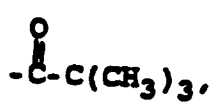
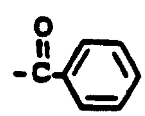

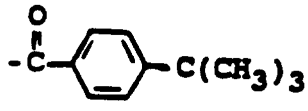
or
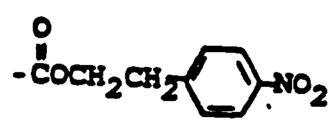

or

B is

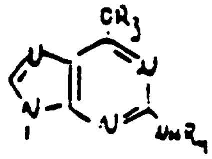, 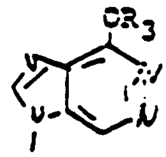 or 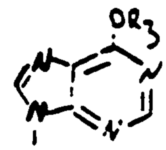

with $R^3$ being, in each case independently of one another, hydrogen or

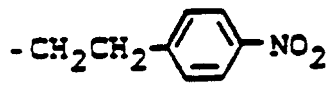

and

$R^4$ being, in each case independently of one another, a group of the formula

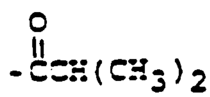, 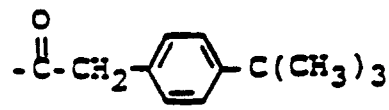 or

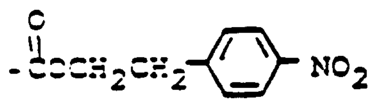

or

B is

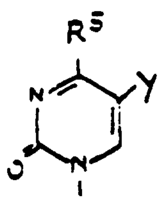 or 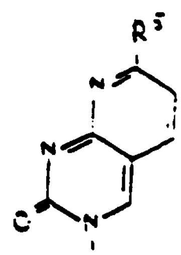

Y = H, $CH_3$ (or: n-alkyl $C_1$-$C_4$) with $R_5$ being, in each case independently of one another, -OH,

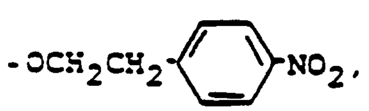,

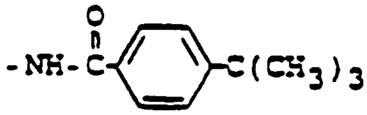 or 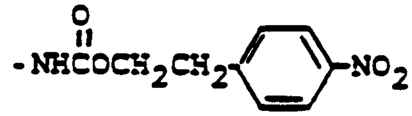

**3.** A process for the preparation of a compound of the formula (IIIa) or (IIIb) by reacting the compound of the formula (I) with an appropriate compound of the formula (IVa) or (IVb)

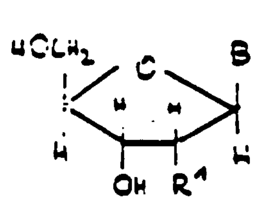 (IVa) 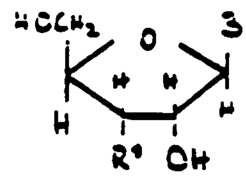 (IVb)

in which $R^1$ and B have the above meaning, in the presence of a base.

**4.** A compound of the formula (Va) or (Vb)

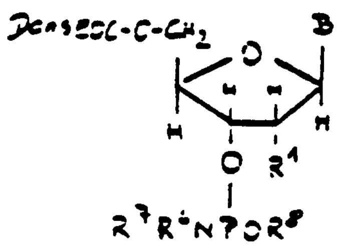 (Va) 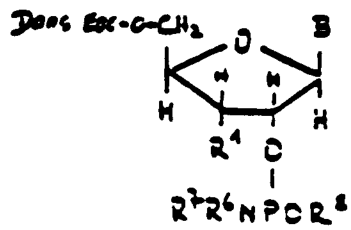 (Vb)

in which
DansEOC, $R^1$ and B have the abovementioned meaning, and $R^6$ and $R^7$ are, identically or independently differently, a $C_1$-$C_8$-alkyl, preferably an isopropyl or $C_5$-$C_{12}$-cycloalkyl group, preferably up to $C_8$, a benzyl or phenyl group or, together with the nitrogen atom to which they are bonded, a saturated or unsaturated heterocyclic ring which can optionally contain further hetero atoms and substituents, and $R^8$ is a group of the formula

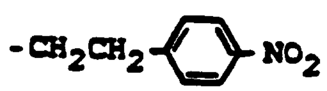, 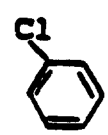

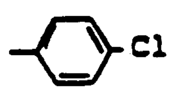

or $CH_3$,
or a benzyl group which is unsubstituted or ring-substituted one or more times, preferably unsubstituted, where the substituent(s) is, independently of one another, a halogen, a $C_1$-$C_4$-alkyl, nitro, methoxy or carboxyl group.

**5.** A process for the preparation of a compound of the formula (Va) or (Vb) by reacting the compound of the formula (IIIa) or (IIIb) with a compound of the formula (VI)

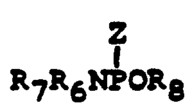 (VI),

in which $R_6$, $R_7$ and $R_8$ have the above meaning, and Z is chlorine or bromine or a radical of the formula $-NR_9R_{10}$ where $R_9$ and $R_{10}$ are, identically or independently differently, a $C_1$-$C_8$-alkyl,

preferably an isopropyl or $C_5$-$C_{12}$-cycloalkyl group, preferably up to $C_8$, a benzyl or a phenyl group, preferably chlorine, in the presence of a base.

**6.** A process for the preparation of oligonucleotides from compounds of the formula (Va) and/or (Vb), which comprises a compound of the formula (Va) or (Vb)

1. being reacted with a compound of the formula (VIIa) or (VIIb)

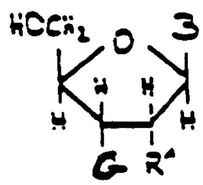 (VIIa) or 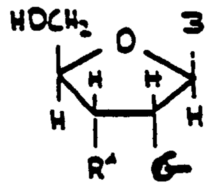 (VIIb)

in which B and $R^1$ have the abovementioned meaning, and G has the same meaning as $R^1$ or is a polymeric support which is bonded via the 2'-hydroxyl or 3'-hydroxyl group of the compound of the formula (VIIa) or (VIIb),

2. the resulting compound being oxidized,
3. the dansylethoxycarbonyl group being eliminated,
4. the resulting compound being reacted with a compound of the formula (Va) or (Vb) and
5. reaction steps 2-4 being repeated up to the required chain length.

**7.** A compound of the formula (VIIIa) or (VIIIb)

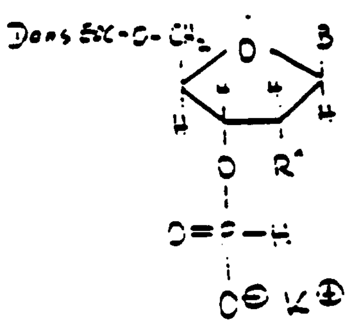 (VIIIa) 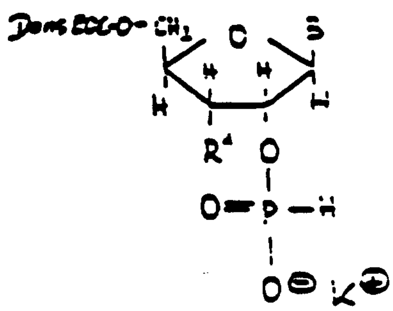 (VIIIb)

in which DansEOC, $R^1$ and B have the abovementioned meaning, and $K^{(+)}$ is a cation, especially $[HN(C_2H_5)_3]^{(+)}$.

**8.** A process for the preparation of a compound of the formula (VIIIa) or (VIIIb) by reacting a compound of the formula (IIIa) or (IIIb) with a compound of the formula (IX)

$PR_{14}R_{15}R_{16}$ (IX)

in which $R_{14}$, $R_{15}$ and $R_{16}$ are, identically or independently differently, hydrogen or a $C_1$-$C_8$-alkyl, $C_1$-$C_8$-fluoroalkyl or aryl group, preferably a 2,2,2-trifluoroethyl, 1,1,1,3,3,3-hexafluoro-2-propyl, ethyl or phenyl group in the presence of a base.

**9.** A process for the preparation of a compound of the formula (VIIIa) or (VIIIb) by reacting a compound of the formula (IIIa) or (IIIb) with a compound of the formula (X)

$PR_{17}R_{18}R_{19}$ (X)

in which $R_{17}$, $R_{18}$ and $R_{19}$ are, identically or independently differently, chlorine, bromine or a $C_1$-$C_8$-alkylamino or a 1,2,4-triazolyl group, preferably a 1,2,4-triazolyl group, in the presence of a base with subsequent hydrolysis.

**10.** A process for the preparation of oligonucleotides from the compounds of the formula (VIIIa) and/or (VIIIb), which comprises a compound of the formula (VIIIa) or (VIIIb)

1. being reacted with a compound of the formula (VIIa) or (VIIb),
2. the dansylethoxycarbonyl group being eliminated,
3. the resulting compound being reacted with a compound of the formula (VIIIa) or (VIIIb),
4. reaction steps 2 and 3 being repeated up to the required chain length and
5. the resulting oligonucleotide being oxidized.

**Claims for the following Contracting State : ES**

**1.** A process for the preparation of the compound of the formula (I),

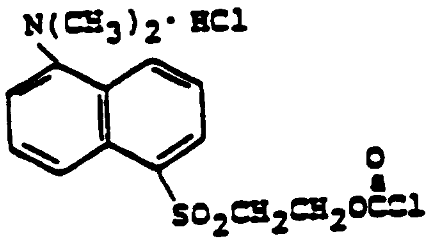

(I),

which comprises reacting the compound of the formula (II),

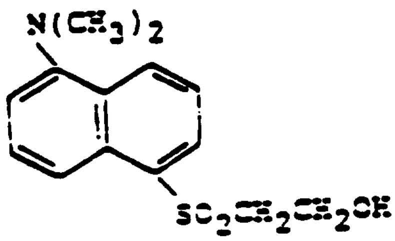

(II),

with a chlorocarbonyl donor.

**2.** A process for the preparation of a compound of the formula (IIIa) or (IIIb)

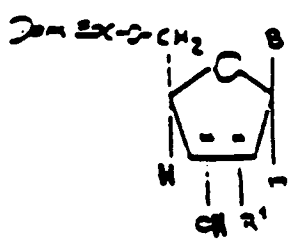

(IIIa)

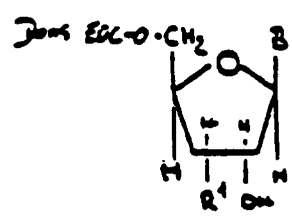

(IIIb)

in which DansEOC is a group of the formula

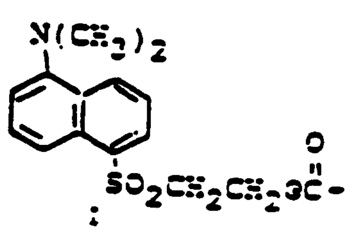

$R^1$ is hydrogen or, independently of one another, a group of the formula

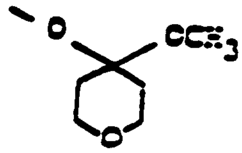

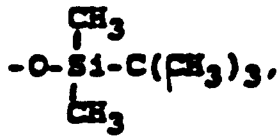

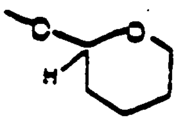

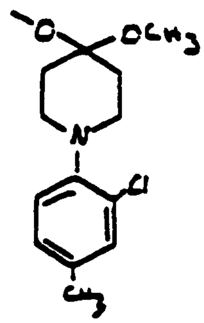

or

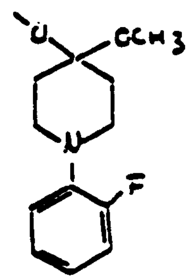

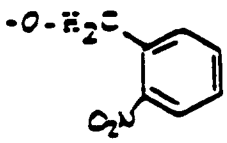

,

and

B is

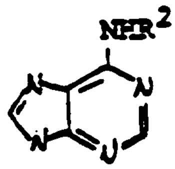

,

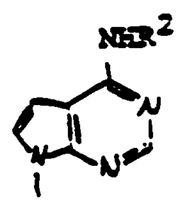

,

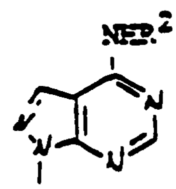

,

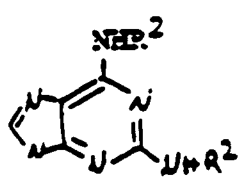

or

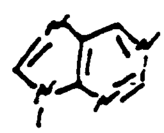

with $R^2$ being, in each case independently of one another, a group of the formula

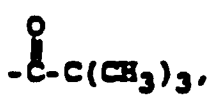

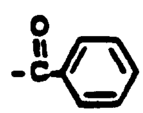

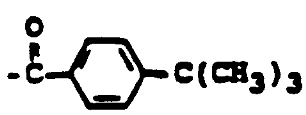

or

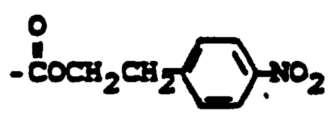

or

B is

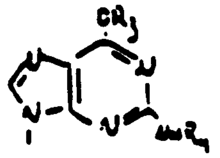, 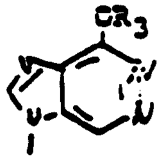 or 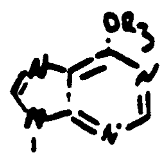

with $R^3$ being, in each case independently of one another, hydrogen or

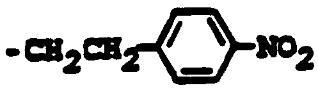

and

$R^4$ being, in each case independently of one another, a group of the formula

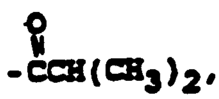, 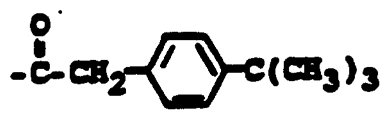 or

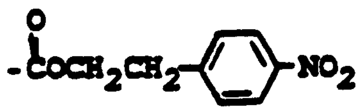.

or

B is

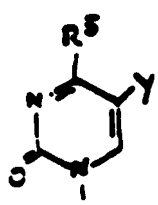 or 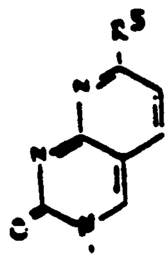

Y = H, $CH_3$ (or: n-alkyl $C_1$-$C_4$) with $R_5$ being, in each case independently of one another, -OH,

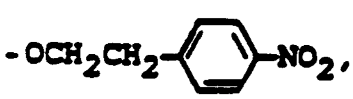,

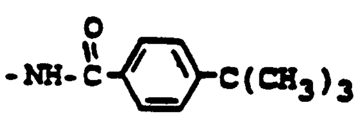 or 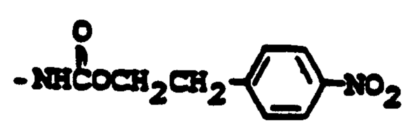,

by reacting the compound of the formula (I) with an appropriate compound of the formula (IVa) or (IVb)

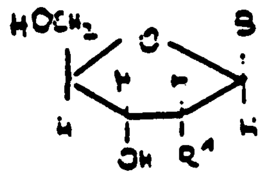 (IVa) 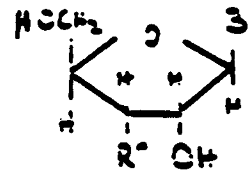 (IVb)

in which $R^1$ and B have the above meaning, in the presence of a base.

**3.** A process for the preparation of a compound of the formula (Va) or (Vb)

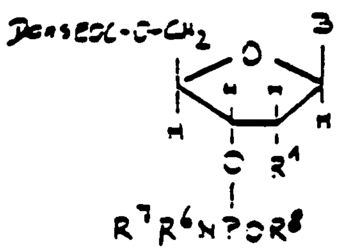 (Va) 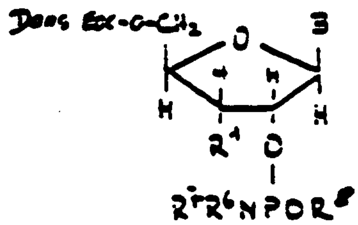 (Vb)

in which
DansEOC, $R^1$ and B have the abovementioned meaning, and $R^6$ and $R^7$ are, identically or independently differently, a $C_1$-$C_8$-alkyl, preferably an isopropyl or $C_5$-$C_{12}$-cycloalkyl group, preferably up to $C_8$, a benzyl or phenyl group or, together with the nitrogen atom to which they are bonded, a saturated or unsaturated heterocyclic ring which can optionally contain further hetero atoms and substituents, and $R^8$ is a group of the formula

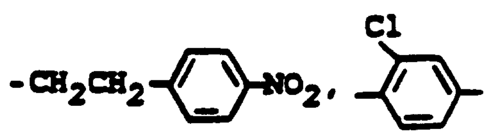

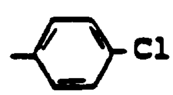

or $CH_3$,
or a benzyl group which is unsubstituted or ring-substituted one or more times, preferably unsubstituted, where the substituent(s) is, independently of one another, a halogen, a $C_1$-$C_4$-alkyl, nitro, methoxy or carboxyl group, by reacting the compound of the formula (IIIa) or (IIIb) with a compound of the formula (VI)

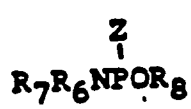 (VI),

in which $R_6$, $R_7$ and $R_8$ have the above meaning, and Z is chlorine or bromine or a radical of the formula $-NR_9R_{10}$ where $R_9$ and $R_{10}$ are, identically or independently differently, a $C_1$-$C_8$-alkyl, preferably an isopropyl or $C_5$-$C_{12}$-cycloalkyl group, preferably up to $C_8$, a benzyl or a phenyl group, preferably chlorine, in the presence of a base.

**4.** A process for the preparation of oligonucleotides from compounds of the formula (Va) and/or (Vb), which comprises a compound of the formula (Va) or (Vb)

1. being reacted with a compound of the formula (VIIa) or (VIIb)

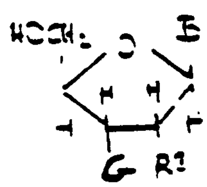

(VIIa) or

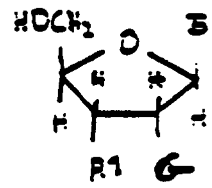

(VIIb)

in which B and $R^1$ have the abovementioned meaning, and G has the same meaning as $R^1$ or is a polymeric support which is bonded via the 2'-hydroxyl or 3'-hydroxyl group of the compound of the formula (VIIa) or (VIIb),

2. the resulting compound being oxidized,
3. the dansylethoxycarbonyl group being eliminated,
4. the resulting compound being reacted with a compound of the formula (Va) or (Vb) and
5. reaction steps 2-4 being repeated up to the required chain length.

**5.** A process for the preparation of a compound of the formula (VIIIa) or (VIIIb)

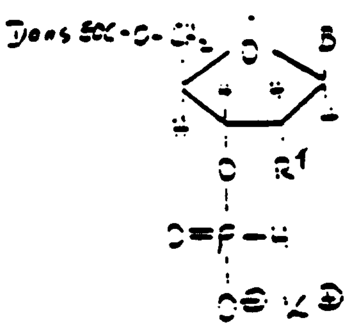

(VIIIa)

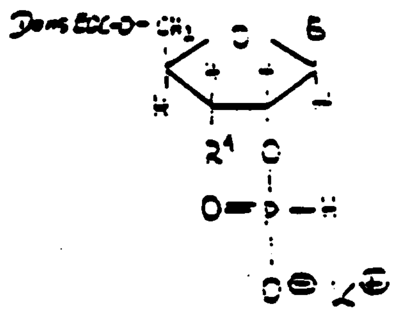

(VIIIb)

in which DansEOC, $R^1$ and B have the abovementioned meaning, and $K^{(+)}$ is a cation, especially $[HN(C_2H_5)_3]^{(+)}$, by reacting a compound of the formula (IIIa) or (IIIb) with a compound of the formula (IX)

$PR_{14}R_{15}R_{16}$ (IX)

in which $R_{14}$, $R_{15}$ and $R_{16}$ are, identically or independently differently, hydrogen or a $C_1$-$C_8$-alkyl, $C_1$-$C_8$-fluoroalkyl or aryl group, preferably a 2,2,2-trifluoroethyl, 1,1,1,3,3,3-hexafluoro-2-propyl, ethyl or phenyl group in the presence of a base.

**6.** A process for the preparation of a compound of the formula (VIIIa) or (VIIIb)

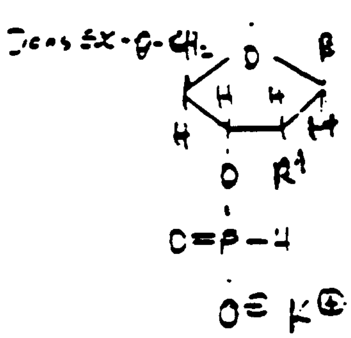

(VIIIa)

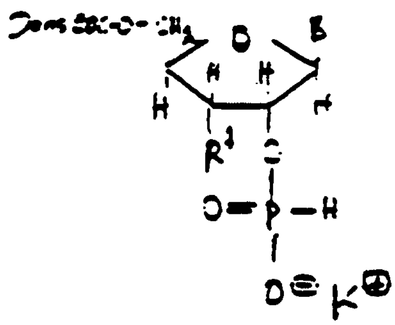

(VIIIb)

in which DansEOC, $R^1$ and B have the abovementioned meaning, and $K^{(+)}$ is a cation, especially $[HN(C_2H_5)_3]^{(+)}$, by reacting a compound of the formula (IIIa) or (IIIb) with a compound of the formula (X)

$PR_{17}R_{18}R_{19}$ (X)

in which $R_{17}$, $R_{18}$ and $R_{19}$ are, identically or independently differently, chlorine, bromine or a $C_1$-$C_8$-alkylamino or a 1,2,4-triazolyl group, preferably a 1,2,4-triazolyl group, in the presence of a base with subsequent hydrolysis.

**7.** The use of a compound of the formulae IIIa, IIIb, Va, Vb, VIIIa or VIIIb in oligonucleotide synthesis.

**8.** A process for the preparation of oligonucleotides and dansylethoxycarbonyl-protected oligonucleotides prepared from at least one compound of the formula (Va) and/or (Vb).

**Revendications**
**Pour les Etats contractants suivants : AT, BE, CH, DE, DK, FR, GB, GR, IT, LI, LU, NL, SE**

**1.** Procédé pour la préparation du composé de formule (I)

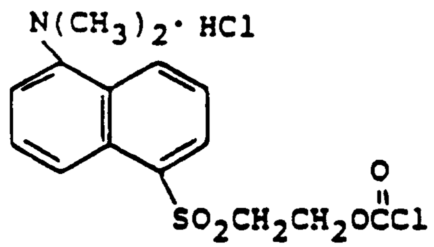

(I)

caractérisé en ce que l'on fait réagir le composé de formule (II)

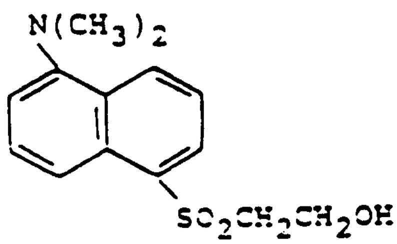

(II)

avec un donneur de groupe chlorocarbonyle.

**2.** Composé de formule (IIIa) ou (IIIb)

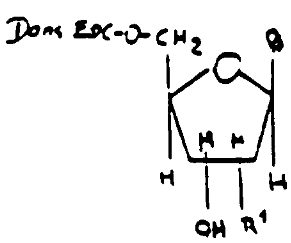

(IIIa)

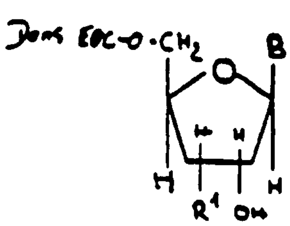

(IIIb)

formules dans lesquelles
Dans-EOC est un groupe de formule

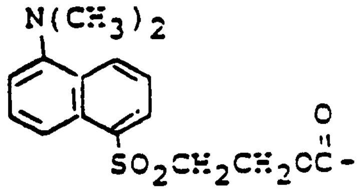

$R^1$ représente un atome d'hydrogène ou, indépendamment l'un de l'autre, un groupe de formule

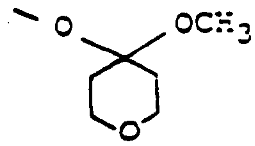

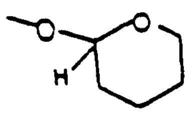 , 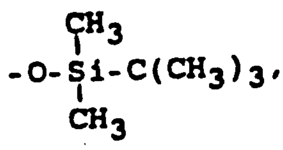

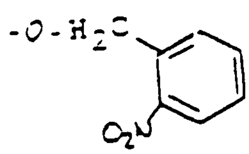 , 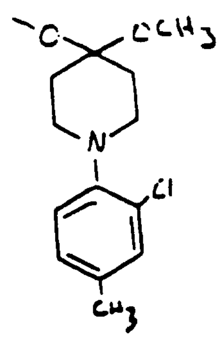 ou 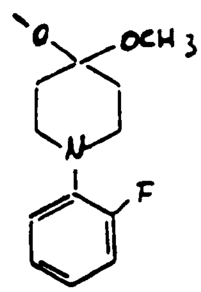

et

B représente

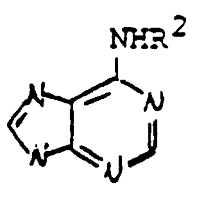 , 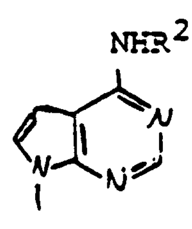 , 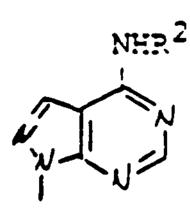 ,

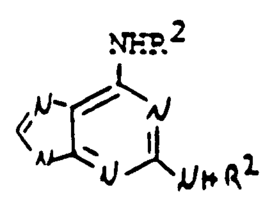 ou 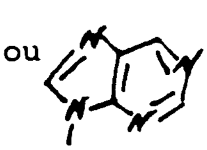

les radicaux $R^2$ représentant chacun, indépendamment l'un de l'autre, un groupe de formule

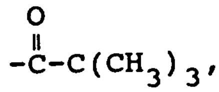 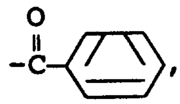

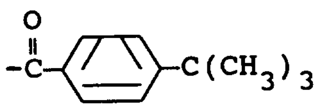 ou 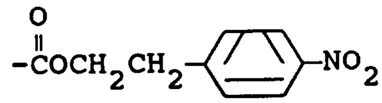

ou

B représente

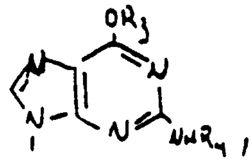 , 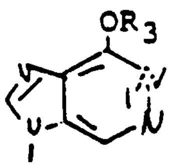 ou 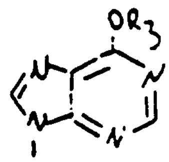

$R^3$ représentant chacun indépendamment les uns des autres un atome d'hydrogène ou

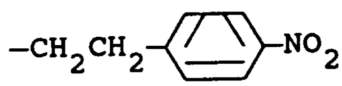

et $R^4$ représentant un groupe de formule

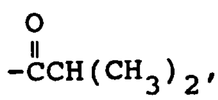

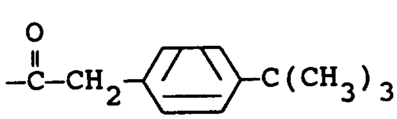 ou 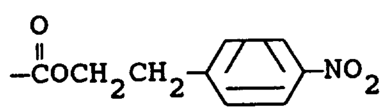

ou

B représente

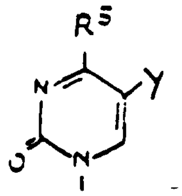 ou 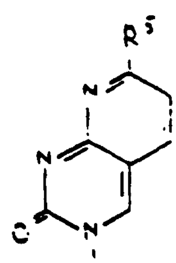

Y = H, $CH_3$ (ou un groupe n-alkyle en $C_1$-$C_4$), $R^5$ étant chacun indépendamment l'un de l'autre

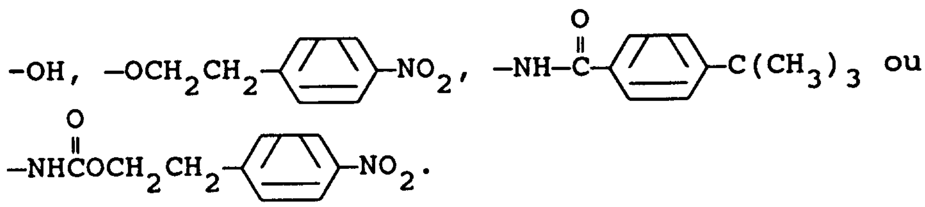

3. Procédé pour la préparation d'un composé de formule (IIIa) ou (IIIb) par la réaction d'un composé de formule (I) avec un composé correspondant de formule (IVa) ou (IVb)

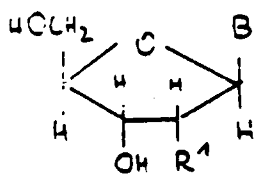
(IVa)

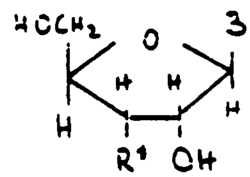
(IVb)

formules dans lesquelles $R^1$ et B ont les significations données plus haut, en présence d'une base.

4. Composé de formule (Va) ou (Vb)

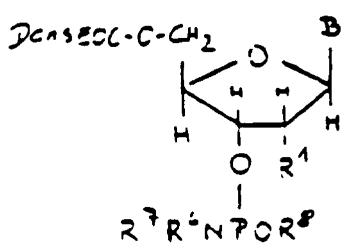
(Va)

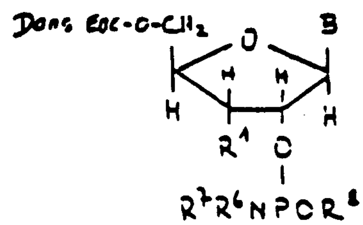
(Vb)

formules dans lesquelles Dans-EOC, $R^1$ et B ont les significations données plus haut, et $R^6$, $R^7$ sont identiques ou différents l'un de l'autre et représentent un groupe alkyle en $C_1$-$C_8$, de préférence un groupe isopropyle, ou cycloalkyle en $C_5$-$C_{12}$, de préférence jusqu'à $C_8$, le groupe benzyle ou phényle ou, conjointement avec l'atome d'azote auquel ils sont liés, un cycle hétérocyclique saturé ou insaturé, qui peut éventuellement contenir d'autres hétéroatomes et substituants, et $R^8$ est un groupe de formule

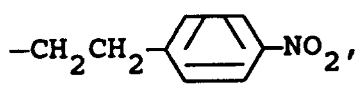

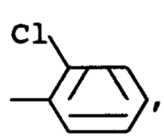,
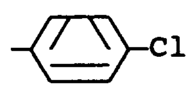

ou $CH_3$, ou un groupe benzyle qui n'est pas substitué au noyau ou est substitué une ou plusieurs fois au noyau, de préférence n'est pas substitué, le ou les substituants étant, indépendamment les uns des autres, un atome d'halogène ou un groupe alkyle en $C_1$-$C_4$, nitro, méthoxy ou carboxy.

5. Procédé pour la préparation d'un composé de formule (Va) ou (Vb), par la réaction du composé de formule (IIIa) ou (IIIb) avec un composé de formule (VI)

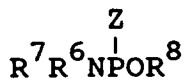 (VI)

dans laquelle $R^6$, $R^7$ et $R^8$ ont les significations données plus haut et Z représente un atome de chlore ou de brome ou un radical de formule $-NR^9R^{10}$, $R^9$ et $R^{10}$ étant identiques ou différents l'un de l'autre, et représentant un groupe alkyle en $C_1$-$C_8$, de préférence un groupe isopropyle, ou cycloalkyle en $C_5$-$C_{12}$, de préférence jusqu'en $C_8$, le groupe benzyle ou phényle, de préférence est un atome de chlore, en présence d'une base.

**6.** Procédé pour la préparation d'oligonucléotides à partir de composés de formule (Va) et/ou (Vb), caractérisé en ce que l'on fait réagir un composé de formule (Va) ou (Vb)

1) avec un composé de formule (VIIa) ou (VIIb)

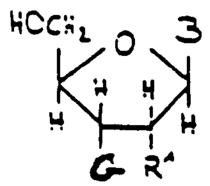 (VIIa) ou 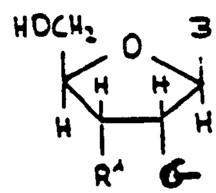 (VIIb)

formules dans lesquelles B et $R^1$ ont les significations données plus haut, et G a la même signification que $R^1$ ou est un support polymère qui est lié par le groupe 2'-hydroxy ou 3'-hydroxy au composé de formule (VIIa) ou (VIIb),

2) on oxyde le composé obtenu,

3) on élimine le groupe dansyléthoxycarbonyle,

4) on fait réagir le composé obtenu avec un composé de formule (Va) ou (Vb) et

5) on répète les étapes de réaction 2 à 4 jusqu'à la longueur de chaîne désirée.

**7.** Composé de formule (VIIIa) ou (VIIIb)

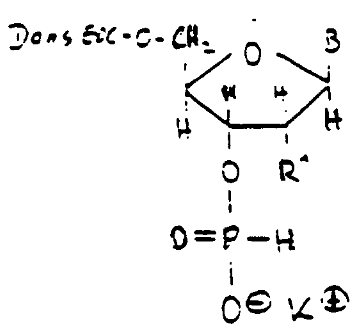 (VIIIa) 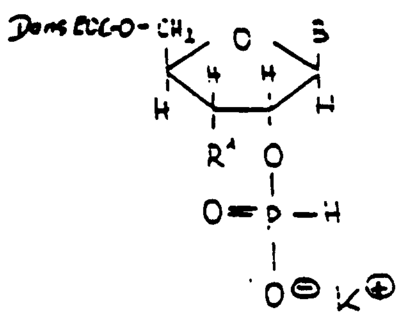 (VIIIb)

formules dans lesquelles Dans-EOC, $R^1$ et B ont les significations données plus haut, et $K^{(+)}$ est un cation, en particulier $[HN(C_2H_5)_3]^{(+)}$.

**8.** Procédé pour la préparation d'un composé de formule VIIIa) ou (VIIIb), par la réaction d'un composé de formule (IIIa) ou (IIIb) avec un composé de formule (IX)

$PR^{14}R^{15}R^{16}$ (IX)

dans laquelle $R^{14}$, $R^{15}$ et $R^{16}$ sont identiques ou différents les uns des autres et représentent un atome d'hydrogène ou un groupe alkyle en $C_1$-$C_8$, fluoroalkyle en $C_1$-$C_8$ ou aryle, de préférence le groupe 2,2,2-trifluoroéthyle, 1,1,1,3,3,3-hexafluoro-2-propyle, éthyle ou phényle, en présence d'une base.

**9.** Procédé pour la préparation d'un composé de formule (VIIIa) ou (VIIIb), par la réaction d'un composé de formule (IIIa) ou (IIIb) avec un composé de formule (X)

$PR^{17}R^{18}R^{19}$ (X)

dans laquelle $R^{17}$, $R^{18}$ et $R^{19}$ sont identiques ou différents les uns des autres et représentent un atome de chlore ou de brome ou un groupe alkyl($C_1$-$C_8$)-amino ou 1,2,4-triazolyle, de préférence le groupe 1,2,4-triazolyle, en présence d'une base, avec hydrolyse subséquente.

**10.** Procédé pour la préparation d'oligonucléotides à partir de composés de formule (VIIIa) et/ou (VIIIb), caractérisé en ce que
1) on fait réagir un composé de formule (VIIIa) ou (VIIIb) avec un composé de formule (VIIa) ou (VIIb),
2) on élimine le groupe dansyléthoxycarbonyle,
3) on fait réagir le composé obtenu avec un composé de formule (VIIIa) ou (VIIIb),
4) on répète les étapes de réaction 2 et 3 jusqu'à la longueur de chaîne désirée, et
5) on oxyde l'oligonucléotide obtenu.

**Revendications pour l'Etat contractant suivant : ES**

**1.** Procédé pour la préparation du composé de formule (I)

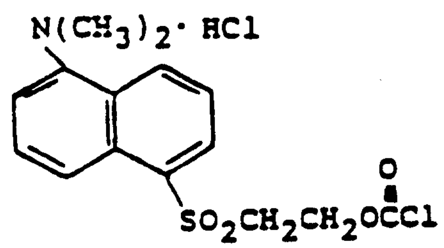

(I)

caractérisé en ce que l'on fait réagir le composé de formule (II)

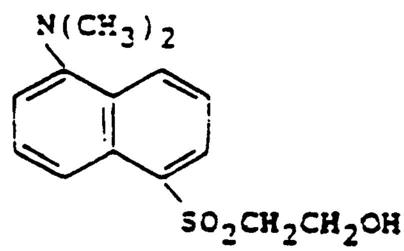

(II)

avec un donneur de groupe chlorocarbonyle.

**2.** Procédé pour la préparation d'un composé de formule (IIIa) ou (IIIb)

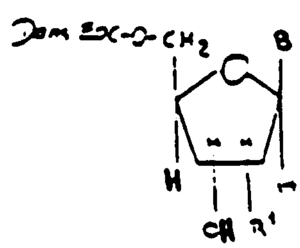

(IIIa)

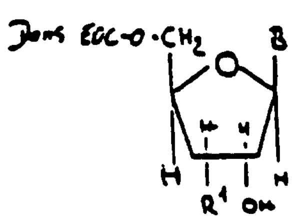

(IIIb)

formules dans lesquelles

Dans-EOC est un groupe de formule

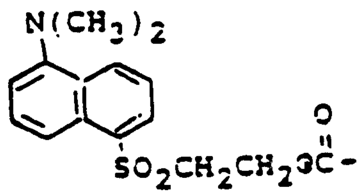

$R^1$ représente un atome d'hydrogène ou, indépendamment l'un de l'autre, un groupe de formule

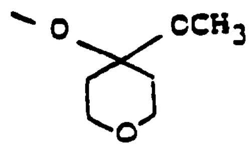

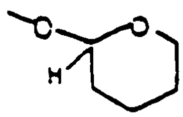

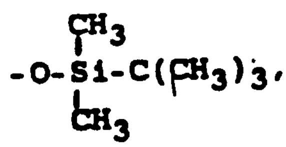

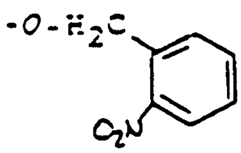

,

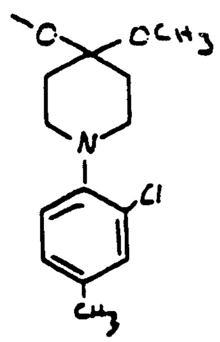

ou

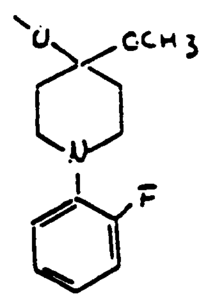

et

B représente

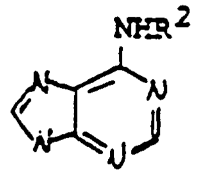 , 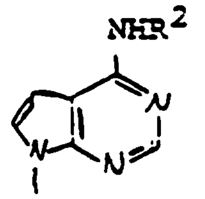 , 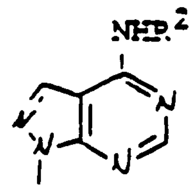 ,

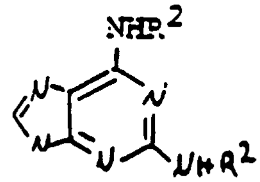 ou 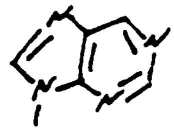

les radicaux $R^2$ représentant chacun, indépendamment l'un de l'autre, un groupe de formule

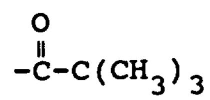 , 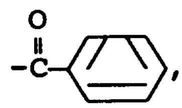 ,

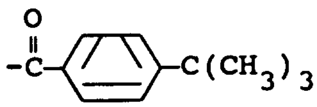 ou 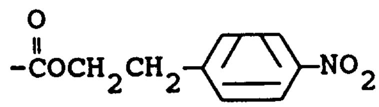

ou

B représente

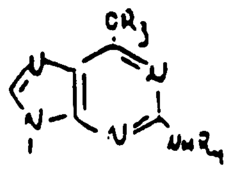 , 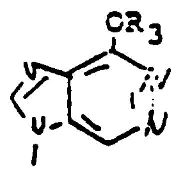 ou 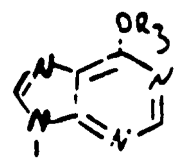

$R^3$ représentant chacun indépendamment les uns des autres un atome d'hydrogène ou

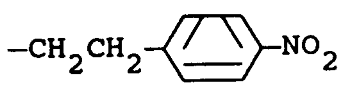

et

$R^4$ représentant un groupe de formule

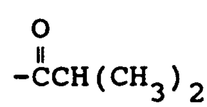 ,

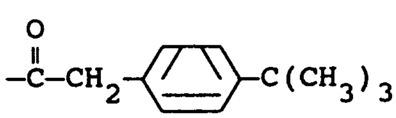 ou 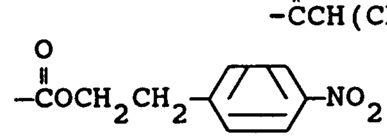

ou

B représente

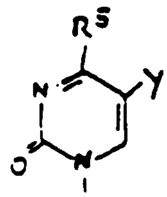

ou

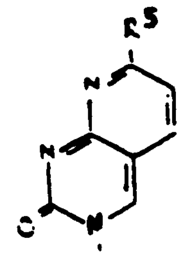

Y = H, $CH_3$ (ou un groupe n-alkyle en $C_1$-$C_4$), $R^5$ étant chacun indépendamment l'un de l'autre

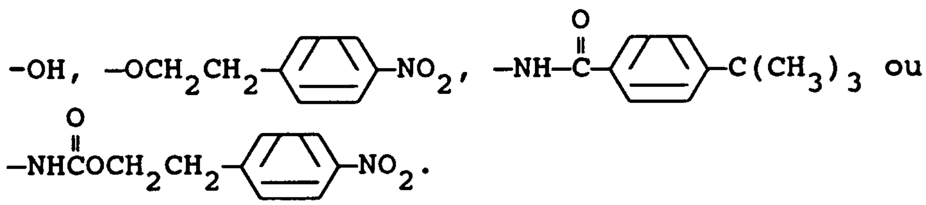

ou

par la réaction du composé de formule (I) avec un composé correspondant de formule (IVa) ou (IVb)

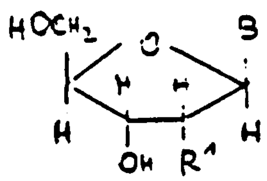

(IVa)

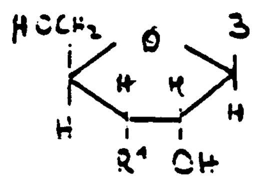

(IVb)

formules dans lesquelles $R^1$ et B ont les significations données plus haut, en présence d'une base.

3. Procédé pour la préparation d'un composé de formule (Va) ou (Vb)

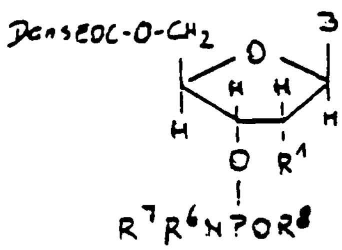

(Va)

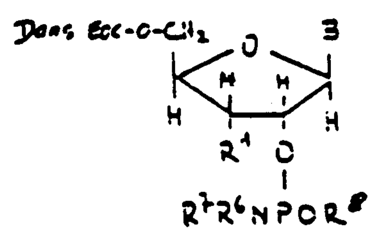

(Vb)

formules dans lesquelles Dans-EOC, $R^1$ et B ont les significations données plus haut, et $R^6$, $R^7$ sont identiques ou différents l'un de l'autre et représentent un groupe alkyle en $C_1$-$C_8$, de préférence un groupe isopropyle,ou cycloalkyle en $C_5$-$C_{12}$, de préférence jusqu'à $C_8$, le groupe benzyle ou phényle ou, conjointement avec l'atome d'azote auquel ils sont liés, un cycle hétérocyclique saturé ou insaturé, qui peut éventuellement contenir d'autres hétéroatomes et substituants, et $R^8$ est un groupe de formule

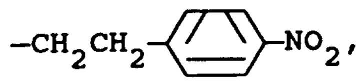

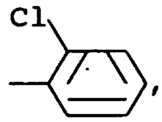, 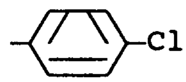

ou $CH_3$, ou un groupe benzyle qui n'est pas substitué au noyau ou est substitué une ou plusieurs fois au noyau, de préférence n'est pas substitué, le ou les substituants étant, indépendamment les uns des autres, un atome d'halogène ou un groupe alkyle en $C_1$-$C_4$, nitro, méthoxy ou carboxy, par la réaction d'un composé de formule (IIIa) ou (IIIb) avec un composé de formule (VI)

$$R^7R^6N\overset{Z}{\overset{|}{P}}OR^8 \qquad (VI)$$

dans laquelle $R^6$, $R^7$ et $R^8$ ont les significations données plus haut et Z représente un atome de chlore ou de brome ou un radical de formule $-NR^9R^{10}$, $R^9$ et $R^{10}$ étant identiques ou différents l'un de l'autre, et représentant un groupe alkyle en $C_1$-$C_8$, de préférence un groupe isopropyle, ou cycloalkyle en $C_5$-$C_{12}$, de préférence jusqu'en $C_8$, le groupe benzyle ou phényle, de préférence est un atome de chlore, en présence d'une base.

4. Procédé pour la préparation d'oligonucléotides à partir de composés de formule (Va) et/ou (Vb), caractérisé en ce que l'on fait réagir un composé de formule (Va) ou (Vb)
   1) avec un composé de formule VIIa) ou (VIIb)

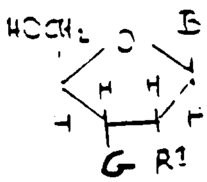 (VIIa) ou 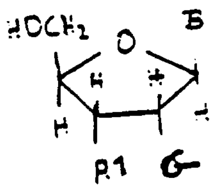 (VIIb)

formules dans lesquelles B et $R^1$ ont les significations données plus haut, et G a la même signification que $R^1$ ou est un support polymère qui est lié par le groupe 2'-hydroxy ou 3'-hydroxy au composé de formule (VIIa) ou (VIIb),
   2) on oxyde le composé obtenu,
   3) on élimine le groupe dansyléthoxycarbonyle,
   4) on fait réagir le composé obtenu avec un composé de formule (Va) ou (Vb) et
   5) on répète les étapes de réaction 2 à 4 jusqu'à la longueur de chaîne désirée.

5. Procédé pour la préparation d'un composé de formule (VIIIa) ou (VIIIb)

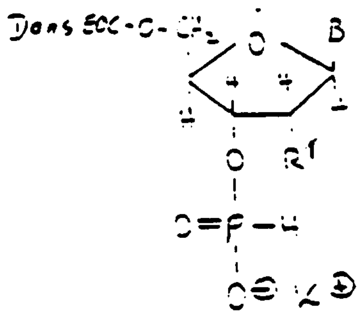

(VIIIa)

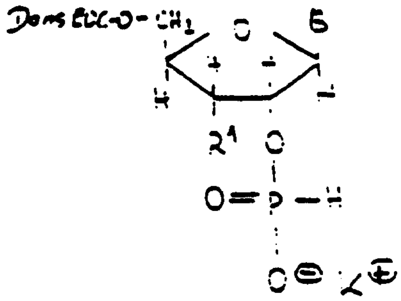

(VIIIb)

formules dans lesquelles Dans-EOC, $R^1$ et B ont les significations données plus haut, et $K^{(+)}$ est un cation, en particulier $[HN(C_2H_5)_3]^{(+)}$,
par la réaction d'un composé de formule (IIIa) ou (IIIb) avec un composé de formule (IX)

$PR^{14}R^{15}R^{16}$ (IX)

dans laquelle $R^{14}$, $R^{15}$ et $R^{16}$ sont identiques ou différents les uns des autres et représente un atome d'hydrogène ou un groupe alkyle en $C_1$-$C_8$, fluoroalkyle en $C_1$-$C_8$ ou aryle, de préférence le groupe 2,2,2-trifluoroéthyle, 1,1,1,3,3,3-hexafluoro-2-propyle, éthyle ou phényle, en présence d'une base.

6. Procédé pour la préparation d'un composé de formule (VIIIa) ou (VIIIb)

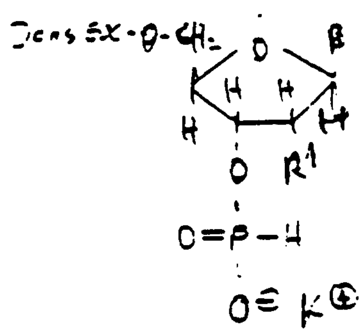

(VIIIa)

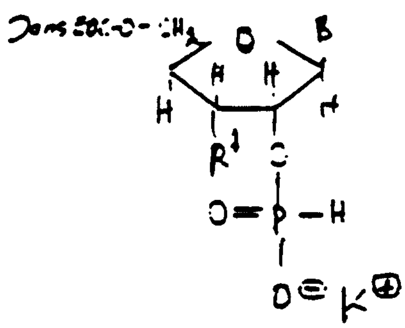

(VIIIb)

formules dans lesquelles Dans-EOC, $R^1$ et B ont les significations données plus haut, et $K^{(+)}$ est un cation, en particulier $[HN(C_2H_5)_3]^{(+)}$,
par la réaction d'un composé de formule (IIIa) ou (IIIb) avec un composé de formule (X)

$PR^{17}R^{18}R^{19}$ (X)

dans laquelle $R^{17}$, $R^{18}$ et $R^{19}$ sont identiques ou différents les uns des autres et représentent un atome de chlore ou de brome ou un groupe alkyl($C_1$-$C_8$)-amino ou 1,2,4-triazolyle, de préférence le groupe 1,2,4-triazolyle, en présence d'une base, avec hydrolyse subséquente.

7. Utilisation d'un composé de formule IIIa, IIIb, Va, Vb, VIIIa ou VIIIb dans la synthèse d'oligonucléotides.

8. Procédé pour la préparation d'oligonucléotides et d'oligonucléotides protégés par le groupe dansyléthoxycarbonyle, préparés à partir d'au moins un composé de formule (Va) et/ou (Vb).